(19) Europäisches Patentamt
European Patent Office
Office européen des brevets

(11) Publication number: 0 021 759 B1

(12) **EUROPEAN PATENT SPECIFICATION**

(45) Date of publication of patent specification: **26.09.84**

(21) Application number: **80302017.1**

(22) Date of filing: **16.06.80**

(51) Int. Cl.³: **C 07 D 263/06,** C 07 D 263/04, A 01 N 25/32 // C07D303/34, C07C93/06, C07C93/04, C07C149/18, C07C149/24

(54) **5-Substituted oxazolidines having herbicide antidote activity, production thereof, herbicidal compositions containing them, use thereof in preventing injury to crops and crop seeds coated therewith.**

(30) Priority: **18.06.79 US 49814**
**18.06.79 US 49676**
**18.06.79 US 49697**

(43) Date of publication of application:
**07.01.81 Bulletin 81/01**

(45) Publication of the grant of the patent:
**26.09.84 Bulletin 84/39**

(84) Designated Contracting States:
**AT BE CH DE FR GB IT LI NL**

(56) References cited:
**US-A-3 959 304**
**US-A-4 072 688**

(73) Proprietor: **STAUFFER CHEMICAL COMPANY**
**Westport Connecticut 06880 (US)**

(72) Inventor: **Teach, Eugene Gordon**
**1929 Downey Place**
**El Cerrito California 94530 (US)**

(74) Representative: **Smith, Sydney et al**
**Elkington and Fife High Holborn House 52/54**
**High Holborn**
**London WC1V 6SH (GB)**

**The file contains technical information submitted after the application was filed and not included in this specification**

EP 0 021 759 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

While many herbicides are immediately toxic to a large number of weed pests, it is known that the effect of many herbicides upon important plant cultivations is either non-selective or not adequately selective. Thus many herbicides damage not only the weeds to be controlled but to a greater or lesser extent, the desirable cultivated plants as well. This holds true for many herbicidal compounds which have been commercially successful and are commercially available. These herbicides include types such as triazines, urea derivatives, halogenated acetanilides, carbamates and thiolcarbamates. Some examples of these compounds are described in U.S. Patents Nos. 2,913,327, 3,037,853, 3,175,897, 3,185,720, 3,198,786, 3,582,314 and 3,952,056.

The side effect of injury to a cultivated crop by various herbicides is particularly inconvenient and unfortunate. When used in the recommended amounts to control broadleaf weeds and grasses, serious malformation or stunting of the crop plants sometimes result. This abnormal growth in the crop plants results in loss of crop yield. The search continues for good selective herbicides.

Previous attempts have been described to overcome this problem. The treatment of the crop seed with certain "hormonal" antagonistic agents prior to planting has been described; see U.S. Patents 3,131,509 and 3,564,768. The protective agents, as well as the herbicide, in these prior processes are largely specific to certain cultivated plant species or in the nature of antagonistic agents. The prior antagonistic agents have not been notably successful. The aforementioned patents specifically exemplify and describe the treatment of seeds employing compounds of a different chemical class, not suggestive of the present invention.

U.S. Patents 3,959,304, 3,989,503, 4,072,688 and 4,124,372 disclose certain substituted oxazolidine compounds. The compounds of the present invention are distinguished from the disclosure of the prior art both from the point of view of structure and utility as herbicidal antidotes for thiolcarbamate herbicides; in particular for S-n-propyl N,N-di-n-propyl thiolcarbamate, S-ethyl di-n-propyl thiocarbamate, S-isopropyl 1-(5-ethyl-2-methylpiperidine) carbothioate, S-ethyl diisobutyl thiolcarbamate, and S-ethyl cyclohexyl ethyl thiolcarbamate.

It has been discovered that cultivated crop plants can be substantially protected against injury by thiol-carbamate-type herbicides, and said injury can be decreased by the use as antidotes of the herein defined compounds in a variety of ways as more fully described below.

The invention provides in one aspect thereof N-haloacyl oxazolidines of the following formula

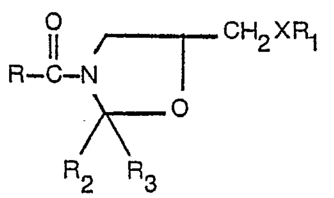

in which

(A) the group $XR_1$ is phenoxy, and R is selected from haloalkyl containing from 1 to 5 carbon atoms, where halo is chloro or bromo, and *p*-methyl phenylsulfonylamido; and $R_2$ and $R_3$ are independently selected from lower alkyl containing from 1 to 3 carbon atoms, where $R_2$ and $R_3$ are lower alkyl this preferably includes those members which contain from 1 to 3 carbon atoms, in particular the following: Methyl, ethyl, n-propyl and isopropyl. Where R is haloalkyl, this preferably includes those members which contain from 1 to 5 carbon atoms, and the term "halo" includes chloro and bromo as mono, di, tri, tetra or hexa substitutions, that is from 1 to 6 halo substituents.

(B) the group X is oxygen or sulphur, $R_1$ is selected from alkyl and alkenyl; R is selected from haloalkyl and alkylthio; and $R_2$ and $R_3$ are independently selected from hydrogen and alkyl containing 1 to 3 carbon atoms, provided that $R_1 + R_2$ is less than or equal to 6 carbon atoms and further provided that when $XR_1$ is thioethyl, then R is other than haloalkyl having 3 or 4 carbons and provided that when $XR_1$ is methoxy, R is other than 2,3-dibromopropyl or the N-haloacyl oxazolidine is one of the following specific compounds 2,2-dimethyl-3(3-bromopropionyl)5-pentoxymethyl oxazolidine; 2,2-dimethyl-3(5-chlorovaleryl)5-isopropoxymethyl oxazolidine; 2,2-dimethyl-3(5-chlorovaleryl)5-allyloxymethyl oxazolidine.

In the above description, the following embodiments are intended for the various substituent groups: when $R_1$ is alkyl this preferably includes those members which contain from 1 to 6 carbon atoms, in both straight chain and branched chain configurations in particular the following: Methyl, ethyl, n-propyl, isopropyl, n-butyl, sec.-butyl, isobutyl, tert.-butyl, and n-hexyl; when $R_1$ is alkenyl this preferably includes those members which contain from 3 to 6 carbon atoms and at least one ethylenic or double bond such as in 1-propenyl, 2-butenyl, 3-butenyl or 1,1-dimethyl-3-butenyl. When $R_2$ and $R_3$ are lower alkyl this preferably includes those members which contains from 1 to 3 carbon atoms, and when R is haloalkyl this preferably includes those members which contain from 1 to 5 carbon atoms, and the term "halo" includes chloro, bromo and fluoro as mono, di, tri, tetra or hexa substitutions, that

is from 1 to 6 halo substituents; and when R is alkylthio this preferably includes those members which contain from 1 to 4 carbon atoms. Other substituent groups are as indicated in carbon content in the above description.

(C) the group X is oxygen or sulphur and $R_1$ is alkyl; R is selected from the group consisting of haloalkyl, and alkoxycarboalkyl; $R_2$ is selected from the group consisting of phenyl, p-chloro-phenyl and p-bromophenyl; and $R_3$ is selected from the group consisting of hydrogen and methyl.

When $R_1$ is alkyl this preferably includes those members which contain from 1 to 6 carbon atoms, in both straight chain and branched chain configurations, in particular the following: Methyl, ethyl, n-propyl, isopropyl, n-butyl, sec.-butyl, isobutyl, and tert.-butyl. When R is haloalkyl this preferably includes those members which contain from 1 to 4 carbon atoms, inclusive, and the term "halo" includes chloro and bromo as mono, di, tri, tetra or hexa substitutions, that is from 1 to 6 halo substituents; and when R is alkoxycarboalkyl this preferably includes those members having 3 to 6 carbon atoms, such as methoxy propanoyl, methoxy ethanoyl (acetyl), ethoxy propanoyl or ethoxy ethanoyl. Other substituent groups are as indicated in carbon content in the above description.

The compounds according to the invention may be formulated in a composition with a thiolcarbamate herbicide and according to a further aspect of the invention there is provided a herbicidal composition which contains a thiolcarbamate herbicide and an antidotally effective amount of an oxazolidine according to the invention.

It seems that the compounds of this invention may interfere with the normal herbicidal action of the thiolcarbamate-type and other herbicides, to render them selective in their action. Associated with the presence of the antidote of the invention, is a decrease in phytotoxicity with respect to various crops, otherwise observed when various thiolcarbamate herbicides are used for weed control. The corresponding beneficial and desirable effect is the continued herbicidal effect of the thiolcarbamate against weed species present in the crop, with the accompanying decreased herbicidal effect on desired crop species. This advantage and utility will become more apparent hereinafter.

Therefore, the terms "antidote", "herbicide antidote" or "antidotal amount" are meant to describe that effect which tends to counteract the normal injurious herbicidal response that the herbicide might otherwise produce. Whether it is to be termed a remedy, interferant, protectant or antagonist, will depend upon the mode of action. The mode of action is varied, but the effect which is desirable, is the result of the method of treating the seed, soil or furrow in which a crop is planted.

The compounds of this invention wherein the definitions are classified under the heading A, B, and C, can in general be prepared from an intermediate of the formula XI which has the formula

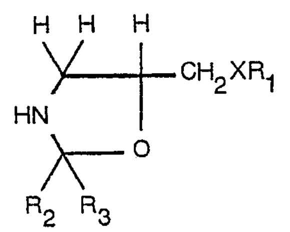

(XI)

in which X, $R_1$, $R_2$ and $R_3$ have the meanings given above as described below.

The general formula covers not only compounds in which X is O but also where X is S. These are represented by formulae VI and X below the production of which will now be described.

*A.* When X is oxygen and $R_1$ is phenyl, the requisite starting material (IV) for the compounds within this invention may be prepared by amination of a 1,2-epoxy-3-phenoxy propane (I), with aqueous ammonia or ammonium hydroxide to produce a 1-amino-3-phenoxy-2-propanol (II). Subsequent reaction and cyclization with acetone or other ketone (III), yields the N-unsubstituted 2,2-dialkyl 5-phenoxymethyl oxazolidine product (IV). This sequence of reactions is depicted by the following equations:

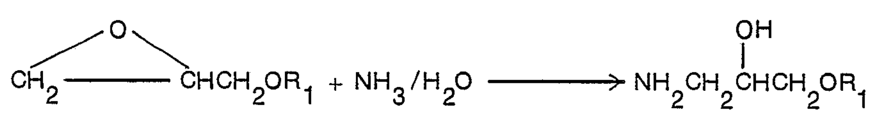

(I) (II)

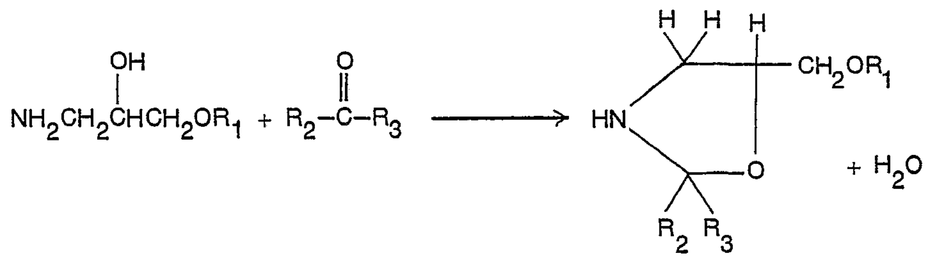

(II) (III) (IV)

wherein

$R_1$, $R_2$ and $R_3$ have the same significance as previously defined.

*B* When X is oxygen and $R_1$ is alkyl or alkenyl, the requisite starting material (IV) for the compounds within this invention may be prepared by amination of a 1,2-epoxy-3-alkoxy or alkenoxy propane (I) with aqueous ammonia or ammonium hydroxide to produce a 1-amino-3-alkoxy or alkenoxy-2-propanol (II). Subsequent reaction and cyclization with acetone or other aldehyde ketone (III) yields the N-unsubstituted 2,2-dialkyl 5-alkoxy or alkenoxy methyl oxazolidine product (IV). This sequence of reactions is depicted by the following equations:

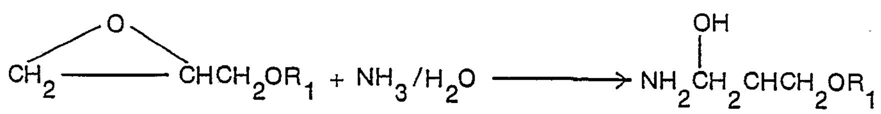

(I) (II)

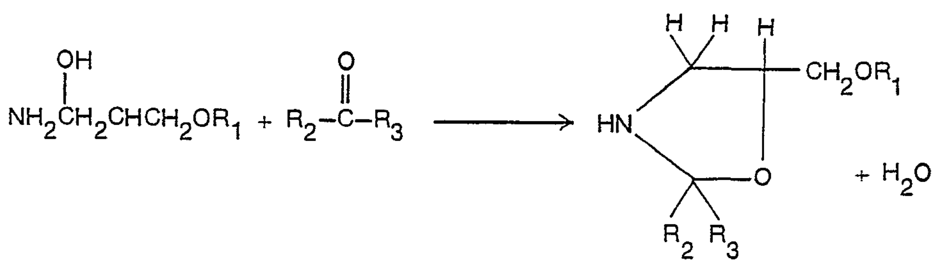

(II) (III) (IV)

wherein

$R_1$, $R_2$ and $R_3$ have the same significance as previously defined.

When X is sulphur and $R_1$ is alkyl or alkenyl, the requisite starting material (X) for the compounds within this invention may be prepared by thionation with a mercaptan (VI) of epichlorohydrin (V) to produce the 1-chloro alkyl or 1-chloro alkenyl thio-2-propanol (VII), this is followed by reformation of the epoxide (VIII). Amination of the 1,2-epoxy-2-alkylthio or alkenylthio propane (VIII) with ammonia or aqueous ammonia produced a 1-amino-3-alkylthio or alkenyl-thio-2-propanol (IX). Subsequent reaction and cyclization with acetone or other aldehyde ketone (III) yields the N-unsubstituted 2,2-dialkyl-5- alkylthio or alkenylthio methyl oxazolidine product (X). This sequence of reactions is depicted by the following equations:

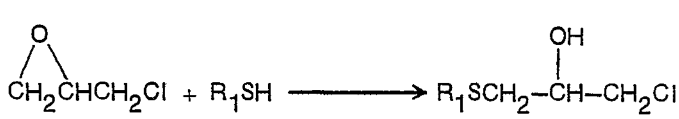

(V) (VI) (VII)

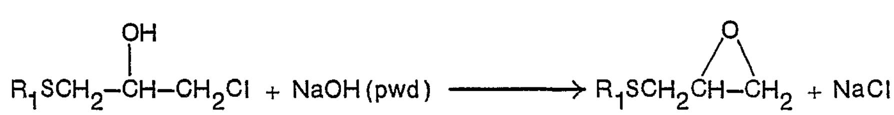

(VII) (VIII)

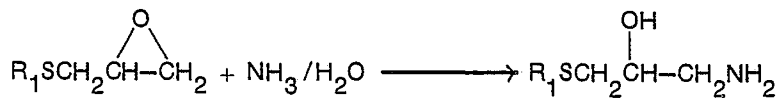

(VIII) (IX)

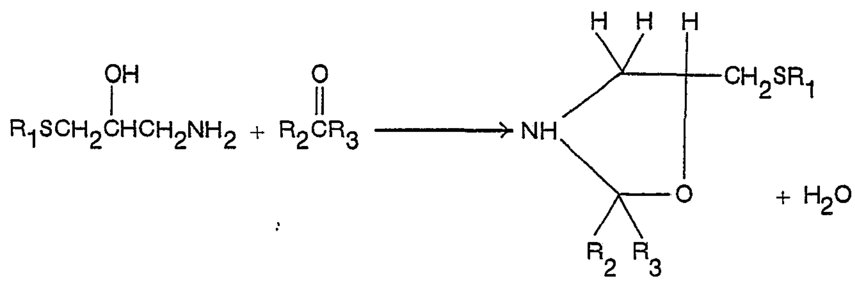

(IX) (X)

*C.* When X is oxygen and $R_1$ is alkyl, the requisite starting material (IV) for the compounds within this invention may be prepared by amination of 1,2-epoxy-3-alkoxy or alkenoxy propane (I) with aqueous ammonia or ammonium hydroxide to produce a 1-amino-3-alkoxy isopropanol (II). Subsequent reaction and cyclization with benzaldehyde or a substituted benzaldehyde, phenyl or substituted phenyl ketone (III), yields the N-substituted 2,2-dialkyl 5-alkoxy oxazolidine product (IV). This sequence of reactions is depicted in the following equations:

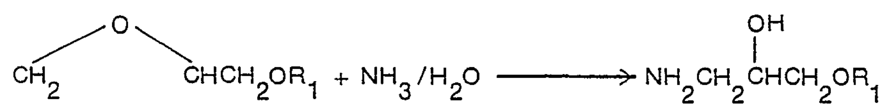

(I) (II)

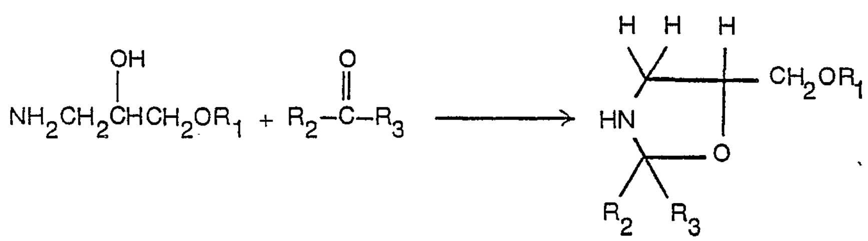

(II) (III) (IV)

wherein

$R_1$, $R_2$ and $R_3$ have the same significance as previously defined.

When X is sulphur and $R_1$ is alkyl, the requisite starting material (X) for the compounds within this invention may be prepared by thionation with a mercaptan (VI) of epichlorohydrin (V) to produce the 1-chloro alkyl thio-2-propanol (VII), this is followed by reformation of the epoxide (VIII). Amination of the 1,2-epoxy-3-alkylthio propane (VIII) with ammonia or aqueous ammonia produce a 1-amino-3-alkylthio 2-propanol (IX). Subsequent reaction and cyclization benzaldehyde or a substituted benzaldehyde, phenyl or substituted phenyl ketone (III) yields the N-unsubstituted 2,2-dialkyl methyl oxazolidine product (X), this sequence of reactions is depicted by the following equations:

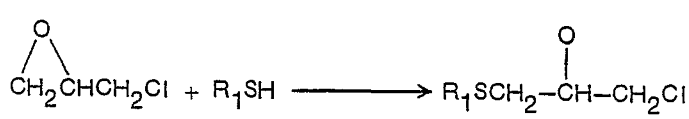

(V) (VI) (VII)

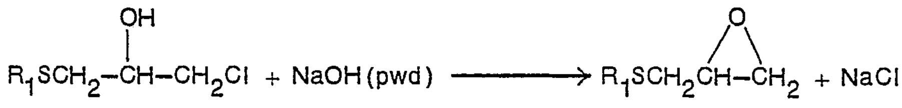

(VII) (VIII)

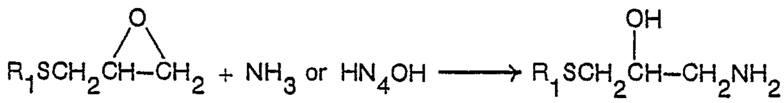

(VIII) (IX)

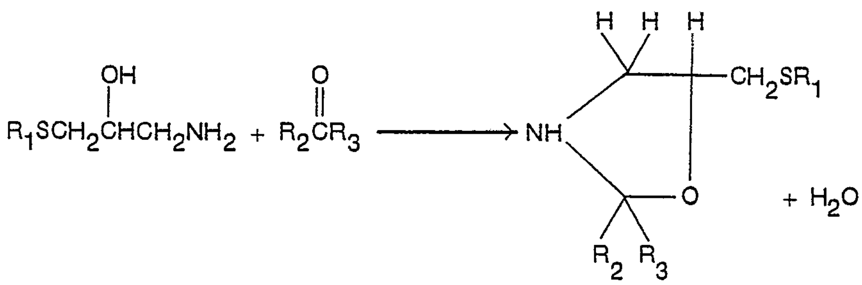

(IX) (X)

The intermediates of formulae (IV) and (X) may then be converted to the end products as follows:—

(a) The N-acyl-substituted compounds of the invention wherein R is haloalkyl or alkoxy carbo alkyl may be prepared by direct acylation of a 5-substituted oxazolidine compound for example with an appropriate acid chloride in the presence of a hydrogen chloride acceptor, such as triethylamine or an inorganic base, such as sodium hydroxide.

(b) The thiocarbamyl 3-substituted compounds of the invention wherein R is alkylthio may be prepared by direct alkylthio formylation of a 5-substituted oxazolidine compound with an alkylhalothioformate e.g. an alkylchlorothioformate suitably in the presence of an acid chloride acceptor.

(c) The p-toluene sulphonylcarbamyl 3-substituted compounds of the invention wherein R is p-toluene sulphonyl may be prepared by direct carbamylation of a 5-substituted oxazolidine compound with p-toluene sulphonyl isocyanate.

In the above reactions, the reaction is preferably performed in the presence of an inert organic solvent, such as benzene. A solvent is normally employed to facilitate the reaction and aid in the work-up of the product. Where good chemical practice dictates a catalyst was used as specified, in some instances a catalyst is not required. The reaction temperatures can vary from —10°C to 90°C. The reaction pressure may be atmospheric, subatmospheric or superatmospheric. However, for convenience of conducting the reactions, the pressure is generally atmospheric. The reaction time will, of course, vary depending upon the reactants and reaction temperature. Generally, the reaction time is from 0.25 to 24 hours. After the reaction is complete, the product is recovered by filtration, extraction and drying. The product can be purified further by trituration with hexane or recrystallization from a suitable solvent. In most instances, the structure was confirmed by analytical techniques, such as infrared spectroscopy, nuclear magnetic resonance or mass spectroscopy.

There is therefore provided according to the invention a process for the preparation of compounds according to the invention which comprises treating an intermediate of the formula

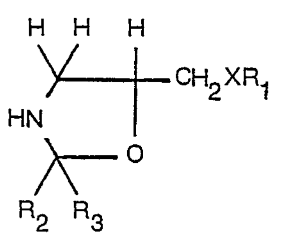

(XI)

in which X, $R_1$, $R_2$ and $R_3$ have the meanings given above and R has the meaning given above by one of the following procedures namely:—

1. For the production of compounds in which R is haloalkyl or alkoxycarboalkyl, direct acylation of the compound of formula XI;

2. For the production of compounds in which R is alkylthio, direct alkylformulation of a compound of formula XI;

3. For the production of compounds in which R is p-toluene sulphonyl direct carbamylation of the compound of formula XI.

In preparing the oxazolidine intermediates it was found that it was unnecessary to isolate and purify the compounds before use. The volume of the oxazolidine solution was adjusted to give a 25 percent w/v solution (4 millilitres — 1 gram) and aliquots were then used for subsequent reactions.

Representative of the above general scheme of reactions, are the following preparations employing specific starting materials and intermediates.

## Preparation of A-Compounds

### Preparation of Intermediate:

1-Amino-3-phenoxy isopropanol

One hundred (100.0) grams of 3-phenoxy-1,2-epoxy propane was added dropwise with stirring to 1 litre of aqueous 28% ammonia cooled to 0°C overnight in the icebox. During addition over 2 hours, a fine precipitate formed and on standing overnight, this formed a layer at the surface of the mixture. The precipitate was taken up in methylene chloride, dried over $MgSO_4$ and stripped. Yield was 58 grams of material mp 69—75°C. A small portion which crystallized from the methylene chloride had mp 79—82°C. The structure was confirmed by infrared and nuclear magnetic resonance.

### Preparation of Intermediate:

1,2-Dimethyl-S-phenoxymethyl oxazolidine

Thirty (30.0) grams of 1-amino-3-phenoxyisopropanol and 15 grams of acetone were combined in 150 millilitres of benzene and refluxed under a modified Dean-Stark apparatus. When about 4 millilitres of water had been removed azeotropically, the mixture was cooled and the volume adjusted to 165.2 millilitres, giving a 25 percent w/v solution (4 millilitres = 1 gram). A small sample was stripped to give the title compound $n_D^{30}$ 1.5150. The structure was confirmed by infrared and nuclear magnetic resonance. Aliquots of this mixture were used in subsequent reactions.

The compounds of the present invention and their preparation are more particularly illustrated by the following examples. Following the examples of preparation is a table of compounds which are prepared according to the procedures described herein. Compound numbers have been assigned to them and are used for identification throughout the balance of the specification.

### Example I

Preparation of 2,2-dimethyl-N-trichloroacetyl-5-phenoxymethyl oxazolidine.

To 24.8 milliliters of 25 percent w/v, 2,2-dimethyl-5-phenoxymethyl oxazolidine in 50 milliliters of benzene was added 5.5 grams of trichloroacetyl chloride. To this solution was added dropwise with cooling 3.1 grams of trimethylamine. After work-up with water, drying and removal of the benzene *in vacuo,* there was obtained 8.1 grams of the title compound, $n_D^{30}$ 1.5272. Analytical data supports the structure.

### Example II

Preparation of 2,2-dimethyl N-2,3-dibromopropionyl-5-phenoxymethyl oxazolidine.

In a similar manner as Example I, to 20.7 milliliters of 25 percent w/v, 2,2-dimethyl-5-phenoxymethyl oxazolidine in 50 milliliters of benzene and 6.6 grams of 2,3-dibromopropional chloride was added 2.6 grams of triethylamine. After the appropriate work-up procedure, there was obtained a yield of 4.3 grams, $n_D^{30}$ 1.5423. Analytical data supports the structure.

### Example III

Preparation of 2,2-dimethyl-3-(p-toluene-sulfonyl carbamyl) 5-phenoxymethyl oxazolidine.

To 20.7 milliliters of 2,2-dimethyl-5-phenoxymethyl oxazolidine, 25 percent w/v in 50 milliliters of benzene was added 419 grams of p-toluene sulfonyl isocyanate. Upon completion of the reaction the solvent, benzene, was removed *in vacuo.* There was obtained 12.8 grams of the title compound, as a glass. Analytical data supports the structure.

Preparation of B-Compounds

Preparation of Intermediate:

1-Chloro-3-ethylthio-2-propanol

Sixty five and two tenths (65.2) grams of ethyl mercaptan was added dropwise with stirring to 92.5 grams of epichlorohydrin and 2 grams of zinc chloride in 500 milliliters of dioxane. The mixture was heated to 40°C and the heating removed while the mixture remained at 35—40°C for 3/4 hour. The mixture was then heated at 40°C for 1 1/2 hour and then to reflux at which point the temperature was 105°C. Yield after stripping was 103 grams, $n_D^{30}$ 1.4862. The product was confirmed by infrared and nuclear magnetic resonance.

Preparation of Intermediate:

3-Ethyl-1,2-epoxypropyl sulfide

One hundred and twenty seven (127.0) grams of 1-chloro-3-ethylthio-2-propanol was added dropwise with vigorous stirring to 82 grams of powdered NaOH in 500 milliliters of diethyl ether. The temperature was kept below 30°C with a water bath and addition took 40 minutes. The product was filtered and the ether stripped off giving 80.8 grams of product, $n_D^{30}$ 1.4567. The structure was confirmed by infrared and nuclear magnetic resonance and used in subsequent reactions without purification.

Preparation of Intermediate:

2-Hydroxy-3-aminopropyl ethyl sulfide

Eighty seven and eight tenths (87.8) grams of 3-ethyl-1,2-epoxypropyl sulfide was added dropwise with stirring to 1 liter of aqueous 28% ammonia solution, cooled to 0°C overnight in an icebox, and maintained in an ice-bath. The epoxide was added over 45 minutes and the reaction mixture was allowed to warm to room temperature and stand in the hood overnight. The water and ammonia were stripped off under vacuum and the oily residue distilled to give 59.1 grams of the title compound; b.p. 101—105°C and 1.5—2 millimeters, $n_D^{30}$ 1.4910. The structure was confirmed by infrared and nuclear magnetic resonance.

In preparing the oxazolidine intermediates it was found that it was unnecessary to isolate and purify the compounds before use. The volume of the oxazolidine solution was adjusted to give a 25 percent w/v solution (4 milliliters = 1 gram) and aliquots were then used for subsequent reactions. Both the 5-oxymethyl and 5-thiomethyl substituted oxazolidines can be prepared by similar reactions.

Preparation of Intermediate:

1-Amino-3-isopropoxy isopropanol

Sixty-three (63.0) grams of 3-isopropoxy-1,2-epoxy propane was added dropwise with stirring to 1 liter of 28 percent aqueous ammonia, cooled to 0°C, and kept in an icebox overnight. The solution was allowed to warm to room temperture and stored in a loosely stoppered container for five days in a hood. The water and ammonia was stripped off under vacuum and the oily residue distilled to give 38 grams of the title compound; b.p. 77.5°C at 0.5 millimeters, $n_D^{30}$ 1.4200. The structure was confirmed by infrared and nuclear magnetic resonance.

Preparation of Intermediate:

2,2-Dimethyl-5-isopropoxymethyl oxazolidine

Twenty six and six tenths (26.6) grams of 1-amino-3-isopropoxy isopropanol and 12 grams of acetone were added to 150 milliliters of benzene and heated to reflux under a modified Dean-Stark apparatus. When about 4 milliliters of water had been azeotropically removed, an additional 20 milliliters of benzene was distilled off and the mixture allowed to cool to room temperature. The volume was adjusted to 138.4 milliliters with benzene to give a 25 percent w/v solution of the title compound. Aliquots of this solution were used to prepare several of the subject compounds.

The compounds of the present invention and their preparation are more particularly illustrated by the following examples. Following the examples of preparation is a table of compounds which are prepared according to the procedures described herein. Compound numbers have been assigned to them and are used for identification throughout the balance of the specification.

Example I

Preparation of 2,2-dimethyl-N-trichloroacetyl-5-isopropoxymethyl oxazolidine.

To 20.8 milliliters of 25 percent w/v, 2,2-dimethyl-5-isopropoxymethyl oxazolidine solution in 50 milliliters of benzene was added 5.5 grams of trichloroacetyl chloride. To this solution was added dropwise with cooling, 3.1 grams of trimethylamine. After washing with water, drying and removal of the benzene *in vacuo,* there was obtained 8.1 grams of the title compound, $n_D^{30}$ 1.4603. Analytical data supports the structure.

Example II

Preparation of 2,2-dimethyl N-dichloroacetyl-5-isopropoxymethyl oxazolidine.

In a similar manner as Example I, 27.7 milliliters of 25 percent w/v, 2,2-dimethyl-5-isopropoxymethyl oxazolidine solution in 50 milliliters of benzene and 4.9 grams of dichloroacetyl chloride was added 4.1 grams of triethylamine. After the appropriate work up procedure, there was obtained a yield of 8.9 grams, $n_D^{30}$ 1.4564. Analytical data supports the structure.

Example III

Preparation of 2,2-dimethyl-3-(p-toluene-sulfonyl carbamyl) 5-isopropoxymethyl oxazolidine.

To 20.8 milliliters of 25 percent w/v, 2,2-dimethyl-5-isopropoxymethyl oxazolidine solution in 50 milliliters of benzene was added 5.9 grams of p-toluene sulfonyl isocyanate. Upon completion of the reaction the solvent, benzene, was removed *in vacuo*. There was obtained 12.8 grams of the title compound, as a glass. Analytical data supports the structure.

Example IV

Preparation of 2,2-dimethyl-3-(2,3-dibromopropionyl)-5-methoxymethyl oxazolidine.

To 11.6 milliliters of 25 percent w/v, 2,2-dimethyl-5-methoxymethyl oxazolidine solution in 50 milliliters of benzene with 5.0 grams of 2,3-dibromopropionyl chloride was added dropwise 2.1 grams of triethylamine. After the reaction was complete the mixture was washed with water, separated, dried and the organic solvent removed *in vacuo*. There was obtained 2.7 grams of the title compound, $n_D^{30}$ 1.4887. Analytical data supports the structure.

Example V

Preparation of 2,2-dimethyl-3-dichloroacetyl-5-allyloxymethyl oxazolidine.

To a reaction mixture of 25 percent w/v, 34.2 milliliters of 2,2-dimethyl-5-allyloxymethyl oxazolidine solution in 100 milliliters benzene with 4 grams of 50% sodium hydroxide solution, was added dropwise, 7.4 grams of dichloroacetyl chloride. Cooling in an ice bath and vigorous stirring was maintained during the addition of the chloride. Upon completion of the reaction the mixture was washed with water, dried, separated and the organic solvent removed *in vacuo.* There was obtained 10.0 grams of the title compound, $n_D^{30}$ 1.4705. Analytical data supports the structure.

Example VI (a—c)

(a) Preparation of 2,2-dimethyl-3-chloroacetyl-5-ethylthiomethyl oxazolidine.

To 21 milliliters of 25 percent w/v, 2,2-dimethyl-5-ethylthiomethyl oxazolidine solution in 25 milliliters of benzene was added dropwise 3.4 grams of chloroacetyl chloride. While cooling in an ice bath and with vigorous stirring, the further dropwise addition of 3.1 grams of triethylamine was carried out. The reaction mixture was allowed to stir for about 1 hour. At the end of this time the mixture was washed with water, separated, dried and the organic solvent removed *in vacuo.* There was obtained 6.2 grams of the title compound, $n_D^{30}$ 1.4942. Infrared data supports the structure.

(b) Preparation of 2,2-dimethyl-3-ethylthiocarbamyl-5-ethylthiomethyl oxazolidine.

To 19.3 milliliters of 25 percent w/v, 2,2-dimethyl-5-ethylthiomethyl oxazolidine solution in 25 milliliters of benzene was added 2.9 grams of triethylamine and 3.4 grams of ethylchlorothiolformate as in the procedure for (a) *supra.* Work-up was similar to (a). There was obtained a yield of 7.1 grams of the title compound, $n_D^{30}$ 1.4940. Infrared data supports the structure.

(c) Preparation of 2,2-dimethyl-3-dichloroacetyl-5-ethylthiomethyl oxazolidine.

To 52.5 milliliters of 25 percent w/v, 2,2-dimethyl-5-ethylthiomethyl oxazolidine solution in 100 milliliters of benzene was added 6.5 grams of 50% solution of sodium hydroxide (10% excess) was added 12.1 grams of dichloroacetyl chloride dropwise. The reaction mixture was cooled in an ice bath with vigorous stirring during the dropwise addition. The temperature was maintained at below 10°C during the addition and allowed to stir about 30 minutes at room temperature. The work-up procedure was similar to (a) *supra.* There was obtained a yield of 15.3 grams of the title compound, $n_D^{30}$ 1.4980.

Preparation of C-Compounds

Preparation of Intermediate:

1-Chloro-3-ethylthio-2-propanol

Sixty five and two tenths (65.2) grams of ethyl mercaptan was added dropwise with stirring to 92.5 grams of epichlorohydrin and 2 grams of zinc chloride in 500 milliliters of dioxane. The mixture was heated to 40°C and the heating removed while the mixture remained at 35—40°C for 3/4 hours. The mixture was then heated at 40°C for 1 1/2 hours and then to reflux at which point the temperature was 105°C. Yield after stripping was 103 grams, $n_D^{30}$ 1.4862. The product was not further purified. The structure was confirmed by infrared and nuclear magnetic resonance spectroscopy.

Preparation of Intermediate:

3-Ethyl-1,2-epoxypropyl sulfide

One hundred and twenty seven (127.0) grams of 1-chloro-3-ethylthio-2-propanol was added dropwise with vigorous stirring to 82 grams of powdered NaOH in 500 milliliters of diethyl ether. The temperature was kept below 30°C with a water bath and addition took 40 minutes. The product was filtered and the ether stripped off giving 80.8 grams of product, $n_D^{30}$ 1.4567. The structure was confirmed by infrared and nuclear magnetic resonance spectroscopy and used in subsequent reactions without purification.

Preparation of Intermediate:

2-Hydroxy-3-aminopropyl ethyl sulfide

Eighty seven and eight tenths (87.8) grams of 3-ethyl-1,2-epoxypropyl sulfide was added dropwise with stirring to 1 liter of aqueous 28 percent ammonia solution, cooled to 0°C overnight in an icebox and maintained in an ice bath. The epoxide was added over 45 minutes and the reaction mixture was allowed to warm to room temperature and stand overnight. The water and ammonia were stripped off under vacuum and the only residue distilled to give 59.1 grams of the title compound, b.p. 101—105°C at 1.4—2 millimeters, $n_D^{30}$ 1.4910. The structure was confirmed by infrared and nuclear magnetic resonance spectroscopy.

In preparing the oxazolidine intermediate it was found that it was unnecessary to isolate and purify the compounds before use. The volume of the oxazolidine solution was adjusted to give a 25% w/v solution (4 milliliters = 1 gram) and aliquots were then used for subsequent reactions. Both the 5-oxymethyl and 5-thiomethyl substituted oxazolidines can be prepared by similar reactions.

Preparation of Intermediate:

1-Amino-3-isopropoxy isopropanol

Sixty three (63.0) grams of 3-isopropoxy-1,2-epoxypropane was added dropwise with stirring to 1 liter of 28 percent aqueous ammonia, cooled to 0°C and kept in an icebox overnight. The solution was allowed to warm to room temperature and stored in a loosely stoppered container for five days in a hood. The water and ammonia was stripped off under vacuum and the only residue distilled to give 38 grams of the title compound, b.p. 77.5°C at 0.5 millimeters, $n_D^{30}$ 1.4200. The structure was confirmed by infrared and nuclear magnetic resonance spectroscopy.

Preparation of Intermediate:

2-p-Chlorophenyl-5-methoxymethyl oxiazolidine

Twelve and six tenths (12.6) grams of 1-amino-3-methoxy-2-propanol and 16.9 grams of p-chlorobenzaldehyde were combined in 150 milliliters of benzene and heated to reflux under a modified Dean-Stark apparatus until about 2.5 milliliters of water were azeotropically removed. An additional 50 milliliters of benzene was distilled over and the mixture was cooled to room temperature and the volume was adjusted to 109.2 milliliters, so that 4 milliliters = 1 gram of intermediate. Aliquots of this solution were used in subsequent reactions.

Preparation of Intermediate:

2-Phenyl-5-ethylthiomethyl oxazolidine

Thirteen and five tenths (13.5) grams of 2-hydroxy-3-aminopropyl ethyl sulfide and 10.6 grams of benzaldehyde were combined in 100 milliliters of benzene and refluxed under a modified Dean-Stark apparatus until about 2 milliliters of water had been azeotropically removed. The mixture was cooled to room temperature and the volume adjusted to 89.2 milliliters (4 milliliters = 1 gram). Aliquots of this mixture were used in subsequent reactions.

The compounds of the present invention and their preparation are more particularly illustrated by the following examples. Following the examples of preparation is a table of compounds which are prepared according to the procedures described herein. Compound numbers have been assigned to them and are used for identification throughout the balance of the specification.

Example I

Preparation of 2-p-chlorophenyl-2(2,3-dibromopropionyl) 5-methoxymethyl oxazolidine.

To 20.8 milliliters of 25 percent w/v, p-chlorophenyl-5-methoxymethyl oxazolidine solution in 50 milliliters of benzene was added 5 grams of 2,3-dibromopropionyl chloride. To this solution was added 1.6 grams of 50% sodium hydroxide. After washing with water, drying and removal of the benzene *in vacuo*, there was obtained 4.8 grams of the title compound, $n_D^{30}$ 1.5478. Analytical data supports the structure.

Example II

Preparation of 2-p-chlorophenyl-3(2,3-dibromopropionyl)5-propoxymethyl.

In a similar manner as Example I, 17.9 milliliters of 25 percent w/v, 2-*p*-chlorophenyl oxazolidine solution in 50 milliliters of benzene and 4.4 grams of 2,3-dibromopropionyl chloride was added 1.4

grams of 50% sodium hydroxide. After the appropriate work-up procedure, there was obtained a yield of 6.4 grams of the title compound, $n_D^{30}$ 1.5310. Analytical data supports the structure.

Example III

Preparation of 2-p-chlorophenyl-3(3-carbomethoxypropionyl)5-propoxymethyl oxazolidine.

To 25.6 milliliters of 25 percent w/v, 2-*p*-chlorophenyl-5-propoxymethyl oxazolidine solution in 50 milliliters of benzene was added 3.8 grams of 3-carbomethoxypropionyl chloride. To this reaction mixture was added 2 grams of 50% sodium hydroxide. Upon completion of the reaction solvent, benzene, was removed *in vacuo*. There was obtained 6.0 grams of the title compound, $n_D^{30}$ 1.4975. Analytical data supports the structure.

Example IV

Preparation of 2-phenyl-2(3-carbomethoxypropionyl)5-methoxymethyl oxazolidine.

To 19.3 milliliters of 25 percent w/v, 2-phenyl-5-methoxymethyl oxazolidine solution in 50 milliliters of benzene with 3.8 grams of 3-carbomethoxypropionyl chloride was added 2.0 grams of 50% sodium hydroxide. After the reaction was complete the mixture was washed with water, separated, dried and the organic solvent removed *in vacuo*. There was obtained 6.8 grams of the title compound, $n_D^{30}$ 1.4913. Analytical data supports the structure.

Example V

Preparation of 2-phenyl-3-chloroacetyl-5-ethylthiomethyl oxazolidine.

To a reaction mixture of 10.1 milliliters of 25 percent w/v, 2-phenyl-5-ethylthiomethyl oxazolidine solution in 25 milliliters benzene, 2.5 grams of triethylamine were added dropwise. Cooling in an ice bath and vigorous stirring was maintained during the addition of the chloride and triethylamine, then allowed to stir one hour at room temperature. Upon completion of the reaction, the mixture was washed with water, dried, and the solvent removed *in vacuo*. There was obtained a yield of 6.0 grams of the title compound, $n_D^{30}$ 1.5410.

TABLE I

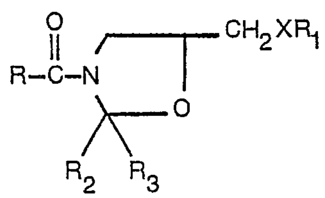

A-Compounds

| Compound Number | R | $XR_1$ | $R_2$ | $R_3$ | Physical Constant $n_D^{30}$ |
|---|---|---|---|---|---|
| 1 | $Cl_3C$— | phenoxy | $CH_3$ | $CH_3$ | 1.5272 |
| 2 | $Cl_2CH$— | phenoxy | $CH_3$ | $CH_3$ | 1.5203 |
| 3 | $ClCH_2$— | phenoxy | $CH_3$ | $CH_3$ | 1.5198 |
| 4 | $CH_3CHBrCHBr$— | phenoxy | $CH_3$ | $CH_3$ | 1.5423 |
| 5 | p-$CH_3$Ø$SO_2NH$— | phenoxy | $CH_3$ | $CH_3$ | (Glass) |
| **B-Compounds** | | | | | |
| 6 | $CCl_3$ | $OCH(CH_3)_2$ | $CH_3$ | $CH_3$ | 1.4603 |
| 7 | $CHCl_2$ | $OCH(CH_3)_2$ | $CH_3$ | $CH_3$ | 1.4564 |
| 8 | $CH_2Cl$ | $OCH(CH_3)_2$ | $CH_3$ | $CH_3$ | 1.4488 |
| 9 | $CH_3CHBrCHBr$ | $OCH(CH_3)_2$ | $CH_3$ | $CH_3$ | 1.4722 |
| 10 | $CH_3$Ø$SO_2NH$ | $OCH(CH_3)_2$ | $CH_3$ | $CH_3$ | (Glass) |

TABLE I (Cont'd)

| Compound Number | R | $XR_1$ | $R_2$ | $R_3$ | Physical Constant $n_D^{30}$ |
|---|---|---|---|---|---|
| 11 | $CHCl_2$ | $OCH_3$ | $CH_3$ | $CH_3$ | 1.4730 |
| 12 | $CH_2BrCHBr$ | $OCH_3$ | $CH_3$ | $CH_3$ | 1.4887 |
| 13 | $C_3H_7S$ | $OCH_3$ | $CH_3$ | $CH_3$ | 1.4620 |
| 14 | $CHCl_2$ | $OC_2H_5$ | $CH_3$ | $CH_3$ | 1.4610 |
| 15 | $CH_2BrCH_2$ | $OC_2H_5$ | $CH_3$ | $CH_3$ | 1.4620 |
| 16 | $CH_2BrCHBr$ | $OC_2H_5$ | $CH_3$ | $CH_3$ | 1.4998 |
| 17 | $CHCl_2$ | $OCH_2CH{=}CH_2$ | $CH_3$ | $CH_3$ | 1.4705 |
| 18 | $CH_2BrCHBr$ | $OCH_2CH{=}CH_2$ | $CH_3$ | $CH_3$ | 1.4934 |
| 19 | $CH_2BrCH_2$ | $OCH_2CH{=}CH_2$ | $CH_3$ | $CH_3$ | 1.4764 |
| 20 | $CH_2ClCH_2$ | $OCH_2CH{=}CH_2$ | $CH_3$ | $CH_3$ | 1.4602 |
| 21 | $CH_3CHCl$ | $OCH_2CH{=}CH_2$ | $CH_3$ | $CH_3$ | 1.4562 |
| 22 | $CHCl_2$ | $OC_3H_7$ | $CH_3$ | $CH_3$ | 1.4560 |
| 23 | $CH_2BrCHBr$ | $OC_3H_7$ | $CH_3$ | $CH_3$ | 1.4795 |
| 24 | $CH_2BrCH_2$ | $OC_3H_7$ | $CH_3$ | $CH_3$ | 1.4612 |
| 25 | $CH_2ClCH_2$ | $OC_3H_7$ | $CH_3$ | $CH_3$ | 1.4470 |
| 26 | $CHCl_2$ | $OC_4H_9$ | $CH_3$ | $CH_3$ | 1.4557 |
| 27 | $CH_2BrCHBr$ | $OC_4H_9$ | $CH_3$ | $CH_3$ | 1.4788 |
| 28 | $CH_2BrCH_2$ | $OC_4H_9$ | $CH_3$ | $CH_3$ | 1.4583 |
| 29 | $CH_2ClCH_2$ | $OC_4H_9$ | $CH_3$ | $CH_3$ | 1.4462 |
| 30 | $CH_3CHCl$ | $OC_4H_9$ | $CH_3$ | $CH_3$ | 1.4420 |
| 31 | $CH_2Cl(CH_2)_3$ | $OCH_3$ | $CH_3$ | $CH_3$ | 1.4588 |
| 32 | $CH_2ClCHBr$ | $OCH_3$ | $CH_3$ | $CH_3$ | 1.4808 |
| 33 | $CH_2ClCHBr$ | $OCH(CH_3)_2$ | $CH_3$ | $CH_3$ | 1.4692 |
| 34 | $CHCl_2$ | $OC_5H_{11}$ | $CH_3$ | $CH_3$ | 1.4508 |
| 35 | $CH_2BrCHBr$ | $OC_5H_{11}$ | $CH_3$ | $CH_3$ | 1.4773 |
| 36 | $CH_2BrCH_2$ | $OC_5H_{11}$ | $CH_3$ | $CH_3$ | 1.4568 |
| 37 | $CH_2Cl$ | $SC_2H_5$ | $CH_3$ | $CH_3$ | 1.4942 |
| 38 | $CH_2BrCHBr$ | $SC_2H_5$ | $CH_3$ | $CH_3$ | 1.5142 |
| 39 | $CH_2BrCH_2$ | $SC_2H_5$ | $CH_3$ | $CH_3$ | 1.4973 |
| 40 | $CH_2ClCH_2$ | $SC_2H_5$ | $CH_3$ | $CH_3$ | 1.4862 |

TABLE I (Cont'd)

| Compound Number | R | $XR_1$ | $R_2$ | $R_3$ | Physical Constant $n_D^{30}$ |
|---|---|---|---|---|---|
| 41 | $C_2H_5S$ | $SC_2H_5$ | $CH_3$ | $CH_3$ | 1.4940 |
| 42 | $CHCl_2$ | $SC_2H_5$ | $CH_3$ | $CH_3$ | 1.4980 |
| 43 | $CH_2Cl(CH_2)_3$ | $OCH_3$ | $CH_3$ | $CH_3$ | 1.4545 |
| 44 | $CH_2Cl(CH_2)_3$ | $OC_2H_5$ | $CH_3$ | $CH_3$ | 1.4509 |
| 45 | $CH_2Cl(CH_2)_3$ | $OC_3H_7$ | $CH_3$ | $CH_3$ | 1.4463 |
| 46 | $CH_2Cl(CH_2)_3$ | $OCH(CH_3)_2$ | $CH_3$ | $CH_3$ | 1.4476 |
| 47 | $CH_2Cl(CH_2)_3$ | $OCH_2CH{=}CH_2$ | $CH_3$ | $CH_3$ | 1.4586 |
| 48 | $CH_2Cl(CH_2)_3$ | $OC_4H_9$ | $CH_3$ | $CH_3$ | 1.4500 |
| 49 | $C_5H_{11}CHBrCHBr$ | $OC_2H_5$ | $CH_3$ | $CH_3$ | 1.4682 |
| 50 | $C_5H_{11}CHBrCHBr$ | $SC_2H_5$ | $CH_3$ | $CH_3$ | 1.4942 |
| 51 | $C_3H_7CHBr$ | $OC_3H_7$ | $CH_3$ | $CH_3$ | 1.4563 |
| C-Compounds | | | | | |
| 52 | $CHCl_2$ | $OCH_3$ | p-Cl phenyl | H | 1.5530 |
| 53 | $CH_2BrCHBr$ | $OCH_3$ | p-Cl phenyl | H | 1.5478 |
| 54 | $CH_2BrCH_2$ | $OCH_3$ | p-Cl phenyl | H | 1.5341 |
| 55 | $CH_2ClCH_2$ | $OCH_3$ | p-Cl phenyl | H | 1.5248 |
| 56 | $CH_3OC(O)CH_2CH_2$ | $OCH_3$ | p-Cl phenyl | H | 1.5094 |
| 57 | $CHCl_2$ | $OC_3H_7$ | p-Cl phenyl | H | 1.5171 |
| 58 | $CH_2BrCHBr$ | $OC_3H_7$ | p-Cl phenyl | H | 1.5310 |
| 59 | $CH_2BrCH_2$ | $OC_3H_7$ | p-Cl phenyl | H | 1.5195 |
| 60 | $CH_2ClCH_2$ | $OC_3H_7$ | p-Cl phenyl | H | 1.5088 |
| 61 | $CH_3OC(O)CH_2CH_2$ | $OC_3H_7$ | p-Cl phenyl | H | 1.4975 |
| 62 | $CHCl_2$ | $OCH_3$ | phenyl | H | 1.5230 |
| 63 | $CH_2ClCH_2$ | $OCH_3$ | phenyl | H | 1.5130 |
| 64 | $CH_3OC(O)CH_2CH_2$ | $OCH_3$ | phenyl | H | 1.4913 |
| 65 | $CH_3CHCl$ | $OCH_3$ | phenyl | H | 1.5075 |
| 66 | $CHCl_2$ | $OC_3H_7$ | phenyl | H | 1.5065 |
| 67 | $CH_2BrCH_2$ | $OC_3H_7$ | phenyl | H | 1.5090 |
| 68 | $CH_2ClCH_2$ | $OC_3H_7$ | phenyl | H | 1.5010 |
| 69 | $CH_3OC(O)CH_2CH_2$ | $OC_3H_7$ | phenyl | H | 1.4868 |

TABLE I (Cont'd)

| Compound Number | R | $XR_1$ | $R_2$ | $R_3$ | Physical Constant $n_D^{30}$ |
|---|---|---|---|---|---|
| 70 | $CH_3CHCl$ | $OC_3H_7$ | phenyl | H | 1.4979 |
| 71 | $CHCl_2$ | $OCH_3$ | p-Br phenyl | H | 1.5363 |
| 72 | $CH_2BrCHBr$ | $OC_3H_7$ | p-Br phenyl | H | 1.5390 |
| 73 | $CH_3OC(O)CH_2CH_2$ | $OC_3H_7$ | p-Br phenyl | H | 1.5032 |
| 74 | $CHCl_2$ | $OC_5H_{11}$ | phenyl | H | 1.4960 |
| 75 | $CH_2BrCHBr$ | $OC_5H_{11}$ | phenyl | H | 1.5123 |
| 76 | $CH_3OC(O)CH_2CH_2$ | $OC_5H_{11}$ | phenyl | H | 1.4940 |
| 77 | $CH_2Cl$ | $SC_2H_5$ | phenyl | H | 1.5410 |
| 78 | $CH_2BrCHBr$ | $SC_2H_5$ | phenyl | H | 1.5580 |
| 79 | $CH_2BrCH_2$ | $SC_2H_5$ | phenyl | H | 1.5461 |

The herbicidal compound employed in the utility of this invention is an active thiolcarbamate herbicide of a general type. That is, it is a member of the class of herbicidally active compounds effective against a wide range of plant species, and may have no discrimination between desirable and undesirable plant species. The method of controlling vegetation comprises applying a herbicidally effective amount of the herein described herbicidal composition to the area or plant, plant locus where control is desired. The herbicidal composition as set forth in this invention include those wherein the antidote is as described above and the preferred active herbicidal compound is selected from the class of thiolcarbamate herbicides and includes the following representative members: S-ethyl dipropyl thiolcarbamate, S-ethyl diisobutyl thiolcarbamate, S-propyl di-n-propyl thiolcarbamate, S-ethyl cyclohexyl ethyl thiolcarbamate, S-ethyl hexahydro-1H-azepine-1-carbothioate, 2,3,3-trichloroallyl N,N-diisopropyl thiolcarbamate, S-isopropyl-1-(5-ethyl-3-methyl-piperidine) carbothioate and S-4-chlorobenzyl diethyl thiolcarbamate.

As an embodiment within the scope of the present invention is a two-part or package herbicide system consisting essentially of a first-part of one or more thiolcarbamate herbicides and a second-part of an antidode compound therefor. It is understood that the antidote compound is used in an effective amount to render the two-part herbicide system selective in decreasing phytotoxic effects to desired or beneficial crops and yet phytotoxic to the undesirable or unwanted vegetation. Thus the soil treated by such a system becomes extremely useful and desirable, allowing previously injured crops to be planted in said treated soil, otherwise injured by the herbicide when used alone. Hence, soil treated with herbicide and antidote as described herein is beneficial, desirable and useful. Likewise, seed treated with the antidote compound is a useful and desirable product.

An herbicide as used herein means a compound which controls or modifies the growth of vegetation or plants. Such controlling or modifying effects include all deviations from natural development; for example, killing, retardation, defoliation, dessication, regulation, stunting, tillering, stimulation, dwarfing and the like. By "plants" it is meant germinant seeds, emerging seedlings and established vegetation including the roots and above-ground portions.

Evaluation Procedure and Method

Flats to be used for growing the crops and weed species were filled with loamy sand soil. Various methods of application were employed, such as pre-plant incorporation (PPI) of 1) the herbicide and antidote separately, and 2) as a tank mix (PPI—TM) with the herbicide and antidote together. The application was by incorporation, whereinafter the seeds of the crops and weeds were planted in the treated soil; application by an in-furrow (IF) treatment of the seeds and surrounding soil in which the herbicide had been applied previously to the soil; and treatment of the crop seeds (ST) with an antidote candidate prior to planting in herbicide treated soil; application to the surface of the soil prior to emergence of the growing plants, (1) as separate application (PES) of herbicide antidote and (2) as a tank mix (PES—TM).

Stock solutions of representative thiolcarbamate herbicides and antidote candidates were prepared as follows:

Herbicides

A. S-ethyl di-n-propyl thiolcarbamate — EPTC—EPTAM® 6E—4133 mg. dissolved in 800 ml. water such that 5 ml. applied to the soil from a planting flat is equivalent to 5 lb/A PPI or 3744 mg. dissolved in 600 ml. of water, 5 ml. of which was equivalent to 6 lb/A PPI

B. S-isopropyl 1-(5-ethyl-2-methyl-piperidine) carbothioate (R—12001) technical, the following is a listing of various stock solutions prepared, also included is the lb/A equivalence per 5 ml. pre-plant incorporated.

120 mg/150 ml acetone; 5 ml = 1 lb/A PPI
176 mg/150 ml acetone; 5 ml = 1.5 lb/A PPI
117 mg/175 ml acetone; 5 ml = 2 lb/A PPI
975 mg/250 ml acetone; 5 ml = 5 lb/A PPI
585 mg/125 ml acetone; 5 ml = 6 lb/A PPI

C. S-ethyl di-isobutyl thiolcarbamate — SUTAN® 6E or S-ethyl cyclohexyl ethyl thiolcarbamate — RONEET® 6E—390 mg. dissolved in 125 ml. water such that 5 ml. applied to the soil from a planting flat is equivalent to 3 lb/A. For 4 lb/A 1456 mg. was dissolved in 350 ml. water, such that 5 ml. was equivalent to the desired amount.

D. S-ethyl hexahydro-1H-azepine-1-carbothioate ORDRAM® 8E—164 mg. dissolved in 75 ml. water such that 5 ml. is equivalent to 2 lb/A applied to the soil from a planting flat pre-plant incorporated.

E. S-propyl di-n-propyl thiolcarbamate — VERNAM® — 6E (80%), the following is a listing of various stock solutions prepared, also included is the lb/A equivalence for 5 ml. pre-plant incorporated:

122 mg/125 ml $H_2O$; 5 ml = 1 lb/A PPI
183 mg/150 ml $H_2O$; 5 ml = 1.25 lb/A PPI
975 mg/250 ml $H_2O$; 5 ml = 4 lb/A PPI
2632 mg/450 ml $H_2O$; 5 ml = 6 lb/A PPI
3412 mg/500 ml $H_2O$; 5 ml = 7 lb/A PPI

Antidotes

F. For each candidate compound employed in the seed treatment method of application, 250 mg. active ingredient was dissolved in 2.5 ml. acetone, with 1% Tween 20® (polyoxyethylene sorbitan monolaurate) such that 0.5 ml. of solution per 10 gm. of seeds is equal to 1/2% w/w.

G. For each candidate compound employed in the "in-furrow" method of application, 95 mg. of active ingredient was dissolved in 15 ml. of acetone with 1% Tween 20®, such that 1.5 ml. applied to the seed and soil in the furrow, in one-half of the flat was equivalent to 5 lb/A. When 1.0 lb/A is desired 0.3 ml. was used.

H. For each candidate compound employed in the "tank mix" pre-plant incorporation test or separately applied pre-plant incorporated test, 50 mg. of active ingredient was dissolved in 100 ml. of acetone with 1% Tween 20®, such that when 10 ml. of the stock solution was further dissolved in 90 ml. of acetone, 4 ml. was equivalent to 1/20 lb/A PPI. When 39 mg. of the compound was dissolved in 10 ml. of acetone, 5 ml. was equivalent to 5 lb /A PPI, and 1 ml. was equivalent to 1 lb/A PPI. When 16 mg. was dissolved in 20 ml., 10 ml. was equivalent to 2 lb/A PPI and when 16 mg. was dissolved in 40 ml., 5 ml. was equivalent to 0 5 lb/A PPI.

In-furrow application of the antidote employed the above stock solutions. As a preparatory step, a one pint sample of soil was removed from each flat to be retained and used later to cover the seeds after treatment with the stock solutions. The soil was leveled before planting. The herbicide stock solution was applied respectively to separate flats and pre-plant incorporated in the soil from the planting flat at the equivalent rate of 1 lb/A active ingredient or the indicated rate.

Rows 1/4-inch (6.36 mm) deep were made lengthwise in each treated flat preparatory to seeding. After seeding, the flats were sectioned into two equal portions using a wooden barrier and 1—1/2 milliliters of additive stock solution was atomized directly onto the exposed seed and soil in the open furrow in one-half of the flat. The untreated section of the flat served as an herbicide check and also made it possible to observe any lateral movement of the antidote through the soil. The seeds were covered with the one-pint sample of untreated soil which had been removed earlier.

For tank mixes to be applied as a pre-plant incorporated application, the following solutions and procedures were employed. Five milliliters (5 ml.) of herbicide stock solutions were each mixed with five milliliters (5 ml.) of antidote candidate stock solution such that the equivalent of 1 lb/A and 5 lb/A of herbicide and antidote, respectively, were applied and incorporated into the soil of each flat. For pre-plant incorporation, the mixed stock solutions were injected into the soil during incorporation in a 5-gallon rotary mixer. Other stock solutions were employed at indicated rates in the tank mix procedure.

In side-by-side tests with various weed species and crops, it was found that weed control was maintained while at the same time the crop species were protected or injury decreased, when compared to a check or control flat. The control flat contained no candidate antidote compound. The following table includes those results.

For seed treatment, 10 grams of seed in a suitable container were shaken with 0.5 milliliters of antidote stock solution H, or other stock solution as indicated, such that the seed treatment was

equivalent to 0.5% w/w, 0.25% w/w, 0.125% w/w or 0.1% w/w. Shaking was continued until the seeds were uniformly covered. The antidote compounds may be applied as liquid slurries and powder or dust treatments. The treated seeds were planted in soil in which herbicide stock solution had been pre-plant incorporated into the soil at a rate equivalent to 1 lb/A active ingredient.

All flats were placed on greenhouse benches where temperatures were maintained between 70—90°F. The soil was watered by sprinkling to assure good plant growth. Injury ratings were taken 2 and 4 weeks after the applications were made. Individual control flats treated with the herbicide alone were included to provide a basis for determining the amount of injury reduction provided by the herbicide antidotes. The results of these tents are tabulated in Table II.

TABLE II

ANTIDOTE ACTIVITY

Application Method:

| | |
|---|---|
| Seed Treatment | ST |
| In-Furrow | IF |
| Pre-Plant Incorporation | PPI |
| Pre-Plant Incorporation-Tank Mix | PPI—TM |

Crop Species:

| | |
|---|---|
| Barley | BA [*Hordeum vulgare* (L.)] |
| Corn | CN [*Zea maize*] |
| Cotton | CT [*Gossypium hirsutum*] |
| Milo (Grain Sorghum) | MO [*Sorghum vulgare*] |
| Rice | RC [*Dryza sativa*] |
| Soybeans | SOY [*Glycine max*] |
| Wheat | WH [*Triticum aestivum*] |

Weed Species:

| | |
|---|---|
| Green Foxtail | FT [*Setaria viridis*] |
| Johnson Grass | JG [*Sorghum halepense*] |
| Nutsedge | NS [*Cyperus esulentus*] |
| Shattercane | SC [*Sorghum bicolor*] |
| Watergrass | WG [*Echinochloa crusgalli*] |
| Wild Oats | WO [*Avena fatua* (L.)] |

$$\text{Result} = \frac{\text{Percent injury with antidote present}}{\text{Percent injury of herbicide alone}}$$

TABLE II (Cont'd)

| Compound Number | Herbicide PPI | Antidote Method of Application | Rate | % Injury | | | |
|---|---|---|---|---|---|---|---|
| | | | | Crop | Result | Weed | Result |
| | | | (Herb. + Anti.) (lbs/A or % ST.) | | | | |
| 1 | VERNAM | PPI—TM | 6+0.5 | SOY | 35/60 | WG<br>FT | 100/100<br>90/90 |
| | VERNAM | PPI—TM | 6+1 | SOY | 40/60 | WG<br>FT | 100/100<br>90/90 |
| | RONEET | IF | 4+5 | WH | 40/60 | FT<br>JG | 80/80<br>100/100 |
| 2 | VERNAM | IF | 6+5 | CN | 10/70 | | |
| | VERNAM | IF | 1+5 | RC | 50/95 | | |
| | R—12001 | PPI | 6+1/80 | CN | 30/50 | WG<br>FT | 95/95<br>95/95 |
| | EPTAM | PPI | 5+5 | CN | 0/80 | WG<br>FT | 100/100<br>100/100 |
| 3 | VERNAM | IF | 1+5 | BA | 30/50 | | |
| | VERNAM | IF | 6+5 | CN | 20/70 | | |
| 4 | VERNAM | IF | 1+5 | WH | 50/70 | | |
| | VERNAM | PPI | 4+5 | SOY | 35/50 | WG<br>FT | 100/100<br>90/90 |
| 5 | VERNAM | IF | 6+5 | SOY | 40/55 | | |
| 6 | VERNAM | IF | 1+5 | CT | 40/70 | | |
| | | IF | 6+5 | CN | 0/70 | | |
| | VERNAM | PPI | 4+1 | SOY | 30/50 | WG<br>FT | 100/100<br>90/90 |
| | EPTAM | PPI—TM | 5+5 | CN | 0/80 | WG<br>FT | 100/100<br>100/100 |
| 7 | VERNAM | IF | 1+5 | MO | 50/95 | | |
| | | IF | 6+5 | CN | 0/70 | | |
| | ORDRAM | ST | 2+0.5% | MO | 10/70 | WG<br>SC | 90/90<br>70/70 |
| | RONEET | ST | 3+0.5% | MO | 10/70 | WG<br>SC | 80/80<br>80/80 |
| | EPTAM | PPI—TM | 5+0.05 | CN | 0/80 | WG | 100/100 |
| | | PPI—TM | 5+5 | CN | 0/80 | FT | 100/100 |
| | R—12001 | ST | 2+0.5% | MO | 50/90 | WG<br>SC | 95/95<br>95/95 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| | RONEET | PPI—TM | 3+1 | MO | 10/80 | WG<br>FT | 100/100<br>100/100 |
| | | | 3+2 | MO | 0/80 | WG<br>FT | 100/100<br>100/100 |
| | | | 3+5 | MO | 0/80 | WG<br>FT | 100/100<br>100/100 |
| 8 | VERNAM | IF | 6+5 | CN | 10/70 | | |
| | RONEET | IF | 3+5 | MO | 40/75 | WG<br>SC | 85/85<br>95/95 |
| 9 | VERNAM | IF | 1+5 | WH | 40/70 | | |
| | | | | CT | 50/70 | | |
| | | | | RC | 30/95 | | |
| | | | | BA | 30/50 | | |
| | | | 6+5 | CN | 30/70 | | |
| | R—12001 | IF | 5+5 | CT | 0/50 | WG<br>FT | 95/95<br>95/95 |
| 10 | VERNAM | IF | 6+5 | SOY | 30/55 | | |
| | | IF | 4+1 | SOY | 20/50 | WG<br>FT | 100/100<br>100/100 |
| | RONEET | IF | 4+5 | MO | 30/60 | FT<br>JG | 80/80<br>100/100 |
| 11 | VERNAM | IF | 1+5 | MO | 50/95 | | |
| | | IF | 6+5 | CN | 0/90 | | |
| | EPTAM | PPI | 5+0.5 | CN | 0/80 | WG<br>FT | 100/100<br>100/100 |
| | | PPI | 5+5 | CN | 0/80 | WG<br>FT | 100/100<br>100/100 |
| | RONEET | IF | 4+5 | MO | 20/60 | FT<br>SC | 80/80<br>100/100 |
| | | PPI—TM | 3+1 | MO | 10/80 | WG<br>FT | 100/100<br>100/100 |
| | | | 3+5 | MO | 0/80 | WG<br>FT | 100/100<br>100/100 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 12 | VERNAM | IF | 1+5 | WH | 50/75 | | |
| | | IF | 6+5 | CN | 0/90 | | |
| | EPTAM | PPI | 5+0.5 | CN | 0/80 | WG<br>FT | 100/100<br>100/100 |
| | | PPI | 5+5 | CN | 0/80 | WG<br>FT | 100/100<br>100/100 |
| 13 | VERNAM | IF | 1+5 | RC | 30/95 | | |
| 14 | VERNAM | IF | 1.25+5 | MO | 10/100 | | |
| | | IF | 1.25+5 | BA | 40/95 | | |
| | | IF | 6+5 | CN | 20/85 | | |
| | RONEET | IF | 3+1 | MO | 40/90 | WG<br>SC | 95/95<br>100/100 |
| | EPTAM | PPI | 5+5 | CN | 0/80 | WG<br>FT | 100/100<br>100/100 |
| 15 | VERNAM | IF | 1.25+5 | MO | 50/100 | | |
| | | | 6+5 | CN | 0/85 | | |
| | RONEET | IF | 3+5 | MO | 30/90 | WG<br>SC | 95/95<br>100/100 |
| | EPTAM | PPI | 5+0.5 | CN | 0/80 | WG<br>FT | 100/100<br>100/100 |
| 16 | VERNAM | IF | 1.25+5 | BA | 30/95 | | |
| 17 | VERNAM | IF | 1.25+5 | MO | 30/100 | | |
| | | IF | 1.25+5 | BA | 30/90 | | |
| | | IF | 6+5 | CN | 0/90 | | |
| | EPTAM | PPI | 5+0.5 | CN | 0/95 | FT<br>WO | 100/100<br>100/100 |
| | RONEET | PPI | 3+1 | MO | 10/90 | WG<br>SC | 100/100<br>100/100 |
| 18 | VERNAM | IF | 1.25+5 | BA | 30/90 | | |
| | | IF | 6+5 | CN | 0/90 | | |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| | EPTAM | PPI | 5+5 | CN | 0/80 | WG<br>FT | 100/100<br>100/100 |
| 19 | VERNAM | IF | 6+5 | CN | 0/90 | | |
| | | IF | 6+5 | SOY | 20/50 | | |
| | EPTAM | PPI | 5+0.5 | CN | 0/80 | WG<br>FT | 100/100<br>100/100 |
| 20 | VERNAM | IF | 6+5 | CN | 0/90 | | |
| | EPTAM | PPI | 5+5 | CN | 0/80 | WG<br>FT | 100/100<br>100/100 |
| 21 | VERNAM | IF | 6+5 | CN | 50/90 | | |
| 22 | VERNAM | IF | 1.25+5 | MO | 30/100 | | |
| | | IF | 6+5 | CN | 0/90 | | |
| | EPTAM | PPI | 5+5 | CN | 0/80 | WG<br>FT | 100/100<br>100/100 |
| | RONEET | PPI | 3+2 | MO | 10/90 | WG<br>SC | 100/100<br>100/100 |
| 23 | VERNAM | IF | 6+5 | CN | 0/90 | | |
| | | IF | 1.25+5 | WH | 60/95 | | |
| | | IF | 1.25+5 | BA | 60/90 | | |
| | EPTAM | PPI | 5+5 | CN | 0/80 | WG<br>FT | 100/100<br>100/100 |
| 24 | VERNAM | IF | 6+5 | CN | 0/90 | | |
| | EPTAM | PPI | 5+0.5 | CN | 0/80 | WG<br>FT | 100/100<br>100/100 |
| 25 | VERNAM | IF | 6+5 | CN | 0/90 | | |
| | EPTAM | PPI | 5+5 | CN | 0/80 | WG<br>FT | 100/100<br>100/100 |
| 26 | VERNAM | IF | 6+5 | CN | 0/90 | | |
| | EPTAM | PPI | 5+5 | CN | 0/80 | WG<br>FT | 100/100<br>100/100 |
| | RONEET | PPI—TM | 3+5 | MO | 0/80 | WG<br>FT | 85/100<br>100/100 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 27 | VERNAM | IF | 6+5 | CN | 0/90 | | |
| | EPTAM | PPI | 5+0.5 | CN | 0/80 | WG<br>FT | 100/100<br>100/100 |
| | VERNAM | IF | 1.25+5 | BA | 60/90 | | |
| 28 | VERNAM | IF | 6+5 | CN | 0/90 | | |
| | EPTAM | PPI | 5+5 | CN | 0/80 | WG<br>FT | 100/100<br>100/100 |
| 29 | VERNAM | IF | 6+5 | CN | 60/90 | | |
| 30 | VERNAM | IF | 6+5 | CN | 60/90 | | |
| 31 | VERNAM | IF | 1.25+5 | MO | 50/100 | | |
| | | IF | 6+5 | CN | 0/90 | | |
| | EPTAM | PPI | 5+5 | CN | 0/70 | WG<br>FT | 100/100<br>100/100 |
| | RONEET | PPI—TM | 3+5 | MO | 40/80 | WG<br>FT | 100/100<br>100/100 |
| 32 | VERNAM | IF | 1.25+5 | MO | 40/100 | | |
| | | IF | 1.25+5 | WH | 50/100 | | |
| | | IF | 1.25+5 | BA | 20/90 | | |
| | | IF | 6+5 | CN | 10/90 | | |
| | RONEET | PPI—TM | 3+5 | MO | 35/80 | WG<br>FT | 100/100<br>100/100 |
| | EPTAM | PPI | 5+5 | CN | 0/70 | WG<br>FT | 100/100<br>100/100 |
| 33 | VERNAM | IF | 1.25+5 | BA | 30/90 | | |
| | | IF | 6+5 | CN | 0/90 | | |
| | EPTAM | PPI | 5+5 | CN | 0/70 | WG<br>FT | 100/100<br>100/100 |
| | VERNAM | PPI—TM | 1.25+5 | WH | 30/80 | WG | 95/95 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 34 | VERNAM | IF | 6+5 | CN | 10/90 | | |
| | SUTAN | PPI—TM | 6+1 | CT | 30/50 | JG<br>NS | 100/100<br>90/90 |
| | EPTAM | PPI | 5+0.5 | CN | 0/70 | WG<br>FT | 100/100<br>100/100 |
| | RONEET | PPI—TM | 3+5 | MO | 15/80 | WG<br>FT | 100/100<br>100/100 |
| 35 | VERNAM | IF | 6+5 | CN | 70/90 | | |
| 36 | VERNAM | IF | 6+5 | CN | 40/90 | | |
| | | IF | 6+5 | SOY | 50/70 | | |
| 37 | VERNAM | IF | 6+5 | CN | 20/90 | | |
| | EPTAM | PPI—TM | 6+5 | CN | 10/85 | WG<br>JG | 98/98<br>98/98 |
| 38 | VERNAM | IF | 1.25+5 | BA | 40/85 | | |
| | | IF | 6+5 | CN | 0/90 | | |
| | EPTAM | PPI—TM | 5+5 | CN | 0/85 | WG<br>FT | 98/98<br>98/98 |
| 39 | VERNAM | IF | 6+5 | CN | 0/90 | | |
| | EPTAM | PPI—TM | 5+0.5 | CN | 0/85 | WG<br>JG | 98/98<br>98/98 |
| 40 | VERNAM | IF | 1.25+5 | BA | 40/85 | | |
| | | IF | 6+5 | CN | 30/90 | | |
| | EPTAM | PPI—TM | 5+5 | CN | 0/85 | WG<br>JG | 98/98<br>98/98 |
| 41 | VERNAM | PPI—TM | 1.25+5 | WH | 50/80 | WG | 50/90 |
| 42 | VERNAM | IF | 6+5 | SOY | 50/65 | | |
| | VERNAM | IF | 1.25+5 | MO | 30/100 | | |
| | | IF | 1.25+5 | BA | 40/85 | | |
| | | IF | 6+5 | CN | 20/90 | | |
| | EPTAM | PPI—TM | 5+0.05 | CN | 0/85 | WG<br>JG | 90/98<br>75/98 |
| | RONEET | PPI—TM | 3+2 | MO | 0/80 | WG<br>FT | 100/100<br>100/100 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 43 | VERNAM | IF | 6+5 | CN | 60/95 | | |
| | | IF | 6+5 | SOY | 50/60 | | |
| 44 | VERNAM | IF | 6+5 | CN | 20/95 | | |
| 45 | VERNAM | IF | 6+5 | SOY | 40/60 | | |
| | | IF | 6+5 | CN | 50/95 | | |
| 46 | VERNAM | IF | 6+5 | CN | 30/95 | | |
| 47 | VERNAM | IF | 6+5 | CN | 35/95 | | |
| | | IF | 6+5 | SOY | 40/60 | | |
| 48 | VERNAM | IF | 6+5 | CN | 75/95 | | |
| 49 | VERNAM | IF | 1.25+5 | BA | 45/75 | | |
| | | IF | 6+5 | SOY | 20/40 | | |
| 50 | VERNAM | IF | 1.25+5 | BA | 50/75 | | |
| 51 | VERNAM | IF | 6+5 | CN | 65/90 | | |
| 52 | VERNAM | IF | 6+5 | CN | 10/90 | | |
| | EPTAM | PPI | 5+5 | CN | 50/70 | WG<br>FT | 100/100<br>100/100 |
| 53 | VERNAM | IF | 1.25+5 | BA | 20/90 | | |
| | VERNAM | IF | 1 1/4+1 | BA | 30/80 | | |
| 54 | VERNAM | IF | 6+5 | SOY | 20/70 | | |
| 55 | VERNAM | IF | 6+5 | SOY | 30/70 | | |
| | | IF | 5+5 | SOY | 30/60 | | |
| 56 | VERNAM | IF | 6+5 | SOY | 30/70 | | |
| | VERNAM | IF | 5+1 | SOY | 20/60 | WG<br>FT | 95/95<br>95/95 |
| | | | 5+5 | SOY | 0/60 | WG<br>FT | 95/95<br>95/95 |
| 57 | VERNAM | IF | 6+5 | CN | 40/90 | | |
| 58 | VERNAM | IF | 1.25+5 | BA | 30/90 | | |
| | | IF | 1.25+1 | BA | 30/80 | FT<br>WO | 70/70<br>95/95 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 59 | VERNAM | IF | 1.25+5 | BA | 75/90 | | |
| 60 | VERNAM | IF | 6+5 | SOY | 60/70 | | |
| 61 | VERNAM | IF | 6+5 | SOY | 10/70 | | |
| | | IF | 5+1 | SOY | 40/60 | WG<br>FT | 95/95<br>95/95 |
| 62 | VERNAM | IF | 6+5 | CN | 0/90 | | |
| | SUTAN | PPI—TM | 6+1 | CT | 10/50 | JG<br>NS | 100/100<br>90/90 |
| | | PPI—TM | 6+5 | CT | 0/50 | JG<br>NS | 100/100<br>90/90 |
| | EPTAM | PPI | 5+0.5 | CN | 50/70 | WG<br>FT | 100/100<br>100/100 |
| | | | 5+5 | CN | 0/70 | WG<br>FT | 100/100<br>100/100 |
| 63 | VERNAM | IF | 6+5 | CN | 80/90 | | |
| 64 | VERNAM | IF | 6+5 | SOY | 30/70 | | |
| | | IF | 5+5 | SOY | 30/60 | WG<br>FT | 95/95<br>95/95 |
| 65 | VERNAM | IF | 6+5 | SOY | 30/70 | | |
| | | IF | 5+5 | SOY | 30/60 | WG<br>FT | 95/95<br>95/95 |
| 66 | VERNAM | IF | 6+5 | CN | 10/90 | | |
| | EPTAM | PPI | 5+5 | CN | 0/70 | WG<br>FT | 100/100<br>100/100 |
| 67 | SUTAN | PPI—TM | 6+1 | CT | 20/50 | JG<br>NS | 100/100<br>90/90 |
| 68 | VERNAM | IF | 6+5 | SOY | 30/70 | | |
| 69 | VERNAM | IF | 6+5 | SOY | 50/70 | | |
| 70 | VERNAM | IF | 6+5 | SOY | 50/70 | | |
| | SUTAN | PPI—TM | 6+5 | CT | 40/50 | JG<br>NS | 100/100<br>90/90 |

TABLE II (Cont'd)

| Compound Number | Herbicide PPI | Antidote Method of Application | Rate | % Injury Crop | Result | Weed | Result |
|---|---|---|---|---|---|---|---|
| | | | (Herb. + Anti.) (lbs/A or % ST.) | | | | |
| 71 | VERNAM | PPI | 1.25+5 | MO | 45/100 | | |
| | RONEET | IF | 3+1 | MO | 40/75 | FT<br>SC | 80/80<br>95/95 |
| | SUTAN | PPI—TM | 6+1 | CT | 40/50 | JG<br>NS | 100/100<br>90/90 |
| | RONEET | PPI—TM | 3+5 | MO | 30/80 | WG<br>FT | 100/100<br>100/100 |
| 72 | SUTAN | PPI—TM | 6+1 | CT | 40/50 | JG<br>NS | 100/100<br>90/90 |
| 73 | VERNAM | IF | 6+5 | SOY | 30/70 | | |
| | VERNAM | IF | 5+5 | SOY | 30/60 | WG<br>FT | 95/95<br>95/95 |
| 74 | VERNAM | IF | 1.25+5 | WH | 80/90 | | |
| 75 | VERNAM | IF | 1.25+5 | WH<br>BA | 80/100<br>75/90 | | |
| 76 | VERNAM | IF | 6+5 | SOY | 40/70 | | |
| 77 | VERNAM | IF | 6+5 | CN | 0/90 | | |
| | EPTAM | PPI—TM | 5+5 | CN | 0/85 | WG<br>JG | 98/98<br>98/98 |
| 78 | VERNAM | IF | 1.25+5 | BA | 30/85 | | |
| | | PPI—TM | 1.25+5 | WH | 60/80 | WG | 90/90 |
| 79 | VERNAM | IF | 1.25+5 | BA | 60/85 | | |
| | | IF | 6+5 | CN | 60/90 | | |

The compounds and compositions of this invention were employed in effective herbicidal compositions comprising the antidote and a thiolcarbamate herbicide as described hereinabove. The herbicidal compositions were tested in the above manner.

A preferred herbicidal compositions consists essentially of a thiolcarbamate herbicide and an antidotally effective amount of an antidote compound therefor corresponding to the formula described hereinabove, and known as 5-oxy or 5-thiomethyl substituted haloacyl oxazolidines.

The compositions of the present invention for the protection of cultivated crop plants comprise the active herbicidal compound and an antidote therefor selected from the above-described compounds. The compositions of herbicide and antidote can be prepared by conventional methods through the thorough mixing and grinding of the active herbicide agents and the antidote with suitable carriers and/or other distribution media, possibly with the addition of dispersion agents or solvents.

The antidote compounds and compositions of the present invention can be used in any convenient form. A solvent or inert carrier is not necessary in view of low volume spray technology which permits the use of neat technical grade materials as sprays. Thus, the antidote compounds and

composition with the thiolcarbamate herbicide can be formulated into emulsifiable liquids, emulsifiable concentrates, liquid, wettable powders, powders, granular or any other convenient form. In its preferred form, a non-phytotoxic quantity of an herbicidal antidote compound is admixed with a selected herbicide and incorporated into the soil prior to or after planting the seed. It is to be understood, however, that the herbicide can be incorporated into the soil. Moreover, the crop seed itself can be treated with a non-phytotoxic quantity of the compound and planted into the soil which has been treated with herbicide, or untreated with the herbicide and subsequently treated with the herbicide. The addition of the antidote compound does not affect the herbicidal activity of the herbicide. The alternative methods of application have been exemplified in the above examples.

The amount of antidote compound present can range between 0.001 to 30 parts by weight of antidote compound described herein per each part by weight of herbicide. The exact amount of antidote compound will usually be determined on economic ratios for the most effective amount usable. It is understood that a non-phytotoxic, but effective quantity of antidote compound will be employed in the herbicidal composition and methods described herein.

After treatment with the antidote and herbicide, there is obtained as a resultant thereof, soil which is novel in composition. Said soil is improved in its capability to grow crops and to offer weed control. Further, said soil treated with herbicide and antidote has the particular utility for allowing seeds of crops otherwise injured by the herbicide, to be planted and grown. The herbicide has its utility in controlling undesirable vegetation; the antidote compound decreases the injury from the herbicide upon the crop species, and the soil treated with herbicide and antidote compound provides an improved media to grow the crop in the presence of an otherwise injurious herbicide.

In the utility of the present antidote compounds and improved herbicide system, the thiolcarbamate can be applied to the soil. Application of the herbicide to the soil can take place by pre-plant incorporation. In conjunction with the prior application of the herbicide employing the present invention crop seeds are planted. Seed planting is followed by application of the antidote as a pre-emergence surface application. This sequence of application of herbicide, seed planting and antidote is unusual and fully effective in decreasing injury to the plant crop, otherwise injured by the thiolcarbamate herbicide.

**Claims for the Contracting States: BE CH DE FR GB IT LI NL**

1. Compounds according to the formula

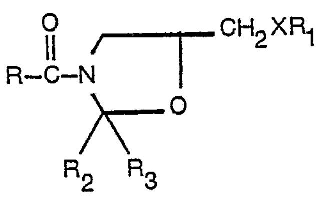

wherein

$XR_1$ is phenoxy, R is selected from haloalkyl containing from 1 to 5 carbon atoms, where halo is chloro or bromo, and p-methyl phenylsulfonylamido; and $R_2$ and $R_3$ are independently alkyl containing from 1 to 3 carbon atoms; or wherein X is oxygen or sulfur, $R_1$ is selected from alkyl and alkenyl; R is selected from haloalkyl and alkylthio; and $R_2$ and $R_3$ are independently selected from hydrogen and alkyl containing from 1 to 3 carbon atoms, provided that $R_1 + R_2$ is less than or equal to 6 carbon atoms and further provided that when $XR_1$ is thioethyl, then R is other than haloalkyl having 3 or 4 carbon atoms and provided that when $XR_1$ is methoxy, R is other than 2,3-dibromopropyl; or wherein X is oxygen or sulphur and $R_1$ is alkyl containing from 1 to 6 carbon atoms; R is selected from haloalkyl containing from 1 to 4 carbon atoms, and the term halo includes chloro and bromo and alkoxy-carbonylalkyl containing 3 to 6 carbon atoms; $R_2$ is selected from phenyl, p-chlorophenyl and p-bromophenyl; and $R_3$ is selected from hydrogen and methyl.

2. Compounds as claimed in claim 1 according to the formula

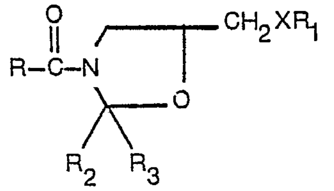

wherein

$XR_1$ is phenoxy, R is selected from haloalkyl containing from 1 to 5 carbon atoms, where halo is chloro or bromo, and p-methyl phenylsulfonylamido; and $R_2$ and $R_3$ are independently selected from alkyl containing from 1 to 3 carbon atoms.

3. Compounds as claimed in claim 1 according to the formula

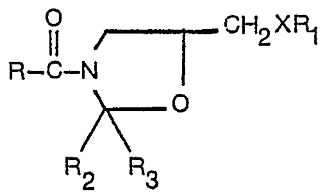

wherein

X is oxygen or sulfur, $R_1$ is selected from alkyl and alkenyl; R is selected from haloalkyl and alkylthio; and $R_2$ and $R_3$ are independently selected from hydrogen and alkyl containing from 1 to 3 carbon atoms, provided that $R_1 + R_2$ is less than or equal to 6 carbon atoms and further provided that when $XR_1$ is thioethyl, then R is other than haloalkyl having 3 to 4 carbons and provided that when $XR_1$ is methoxy, R is other than 2,3-dibromopropyl.

4. Compounds as claimed in claim 1 according to the formula

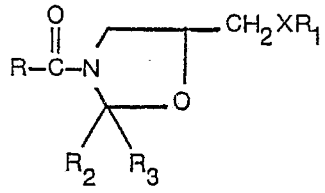

wherein

X is oxygen or sulfur and $R_1$ is alkyl containing from 1 to 6 carbon atoms; R is selected from haloalkyl containing from 1 to 4 carbon atoms, and the term halo includes chloro and bromo and alkoxycarboalkyl containing 3 to 6 carbon atoms; $R_2$ is selected from phenyl, p-chloro-phenyl and p-bromophenyl; and $R_3$ is selected from hydrogen and methyl.

5. Compounds as claimed in claim 1 selected from 2,2-dimethyl-3-(3-bromopropionyl)5-pentoxymethyl oxazolidine, 2,2-dimethyl-3(5-chlorovaleryl)5-isopropoxymethyl oxazolidine, and 2,2-dimethyl-3(5-chlorovaleryl)5-allyloxymethyl oxazolidine.

6. A process for the preparation of a compound as claimed in claim 1 characterised in that it comprises treating an intermediate of the formula

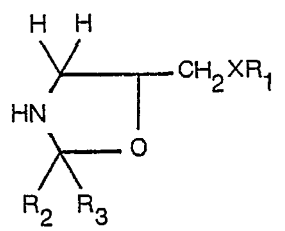

(XI)

in which X, $R_1$, $R_2$ and $R_3$ have the meanings given in claim 1 by one of the following procedures namely:—

1. for the production of compounds in which R is haloalkyl or alkoxycarboalkyl, direct acylation of the compound of formula XI;

2. for the production of compounds in which R is alkylthio, direct alkylthioformylation of a compound of formula XI;

3. for the production of compounds in which R is p-toluene sulfonyl direct carbamylation of the compound of formula XI.

7. A herbicidal composition comprising a thiolcarbamate herbicide and a non-phytotoxic antidotally effective amount of an antidote compound therefor as claimed in claim 1, 2, 3, 4 or 5.

8. The method of decreasing injury to crops, said injury due to a thiolcarbamate herbicide comprising application to the soil in which said crop is to be planted and grown, a non-phytotoxic antidotally effective amount of compound as claimed in claim 1, 2, 3, 4 or 5.

9. A method of decreasing injury to crops, said injury due to a thiolcarbamate herbicide, which comprises applying into the seed furrow to the seed and adjacent soil in the open furrow prior to covering to achieve a planted state, a non-phytotoxic antidotally effective amount of a compound as claimed in claim 1, 2, 3, 4 or 5.

10. A method of protecting a plant crop from injury due to an active herbicidal thiolcarbamate compound comprising applying to the plant seed prior to planting, a non-phytotoxic antidotally effective amount of a compound as claimed in claim 1, 2, 3, 4 or 5.

**Claims for the Contracting State: AT**

1. A process for the preparation of compounds of the formula:—

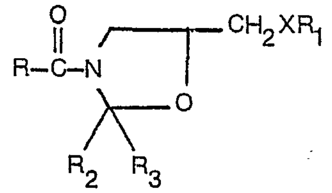

wherein

$XR_1$ is phenoxy, R is selected from haloalkyl containing from 1 to 5 carbon atoms, where halo is chloro or bromo, and p-methyl phenylsulfonylamido; and $R_2$ and $R_3$ are independently selected from alkyl containing from 1 to 3 carbon atoms, or wherein X is oxygen or sulfur, $R_1$ is selected from alkyl and alkenyl; R is selected from haloalkyl and alkylthio; and $R_2$ and $R_3$ are independently selected from hydrogen and alkyl containing from 1 to 3 carbon atoms, provided that $R_1$ and $R_2$ is less than or equal to 6 carbon atoms and further provided that when $XR_1$ is thioethyl, then R is other than haloalkyl having 3 or 4 carbons and provided that when $XR_1$ is methoxy, R is other than 2,3-dibromopropyl; or wherein X is oxygen or sulfur and $R_1$ is alkyl containing from 1 to 6 carbon atoms; R is selected from haloalkyl containing from 1 to 4 carbon atoms, and the term halo includes chloro and bromo and alkoxycarbonylalkyl containing 3 to 6 carbon atoms, $R_2$ is selected from phenyl, p-chlorophenyl and p-bromophenyl; and $R_3$ is selected from hydrogen and methyl which comprises treating an intermediate of the formula

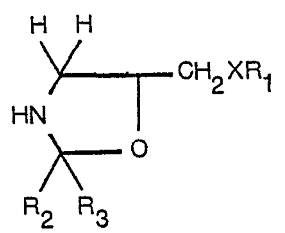

(XI)

in which X, $R_1$, $R_2$ and $R_3$ have the meanings given above by one of the following procedures namely:—

1. for the production of compounds in which R is haloalkyl or alkoxycarboalkyl, direct acylation of the compound of formula XI;

2. for the production of compounds in which R is alkylthio, direct alkylthioformylation or a compound of formula XI;

3. for the production of compounds in which R is p-toluene sulphonyl direct carbamylation of the compound of formula XI.

2. A herbicidal composition comprising a thiolcarbamate herbicide and a non-phytotoxic antidotally effective amount of an antidote compound therefor of formula I defined in claim 1 the preparation of which is claimed in claim 1.

3. The method of decreasing injury to crops, said injury due to a thiolcarbamate herbicide comprising application to the soil in which said crop is to be planted and grown, a non-phytotoxic antidotally effective amount of a compound of formula I defined in claim 1.

4. A method of decreasing injury to crops, said injury due to a thiolcarbamate herbicide, which comprises applying into the seed furrow to the seed and adjacent soil in the open furrow prior to covering to achieve a planted state, a non-phytotoxic antidotally effective amount of a compound of formula I as defined in claim 1, the preparation of which is claimed in claim 1.

5. A method of protecting a plant crop from injury due to an active herbicidal thiolcarbamate compound comprising applying to the plant seed prior to planting, a non-phytotoxic antidotally effective amount of a compound of formula I as defined in claim 1.

**Revendications pour les Etats contractants: BE CH DE FR GB IT LI NL**

1. Composés selon la formule:

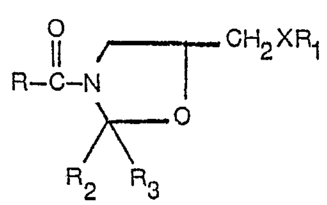

dans laquelle:

$XR_1$ représente un phénoxy;

R est choisi entre un haloalkyle contenant de 1 à 5 atomes de carbone, dans lequel halo représente chloro ou bromo, et un p-méthyl phénylsulfonylamido; et

$R_2$ et $R_3$ sont indépendamment un alkyle contenant de 1 à 3 atomes de carbone; ou dans laquelle:

X représente un oxygène ou un soufre;

$R_1$ est choisi entre un alkyle et un alkényle;

R est choisi entre un haloalkyle et un alkylthio; et

$R_2$ et $R_3$ sont indépendamment choisis entre l'hydrogène et un alkyle contenant de 1 à 3 atomes de carbone, pourvu que $R_1 + R_2$ soient inférieurs à ou égaux à 6 atomes de carbone et pourvu encore que, lorsque $XR_1$ représente un thioéthyle, R est alors autre qu'un haloalkyle possédant de 3 à 4 atomes de carbone et pourvu que, lorsque $XR_1$ représente un méthoxy, R est autre qu'un 2,3-dibromopropyle; ou lorsque X est un oxygène ou un soufre et $R_1$ représente un alkyle contenant de 1 à 6 atomes de carbone, R est choisi entre un haloalkyle contenant de 1 à 4 atomes de carbone, et le terme halo comprend chloro et bromo, et un alkoxycarbonylalkyle contenant de 3 à 6 atomes de carbone; $R_2$ est choisi parmi: phényle, p-chlorophényle et p-bromophényle; et $R_3$ consiste en un hydrogène ou un méthyle.

2. Composés selon la revendication 1, présentant la formule:

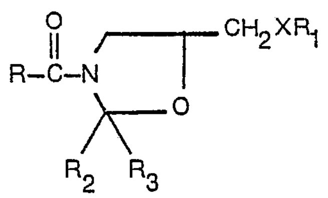

dans laquelle:

$XR_1$ est un phénoxy;

R est choisi entre un haloalkyle contenant de 1 à 5 atomes de carbone, où halo est un chloro ou bromo, et un p-méthylphénylsulfonylamido; et

$R_2$ et $R_3$ sont indépendamment choisis parmi les alkyles contenant de 1 à 3 atomes de carbone.

3. Composés tels que revendiqués dans la revendication 1, selon la formule:

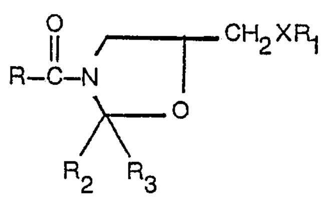

dans laquelle:

X représente un oxygène ou un soufre;

$R_1$ est choisi entre un alkyle et un alkényle;

R est choisi entre un haloalkyle et un alkylthio; et

$R_2$ et $R_3$ sont indépendamment choisis entre un hydrogène et un alkyle contenant de 1 à 3 atomes de carbone, pourvu que $R_1 + R_2$ soient inférieurs à ou égaux à 6 atomes de carbone et pourvu encore que, lorsque $XR_1$ représente un thioéthyle, R est alors autre qu'un haloalkyle possédant 3 ou 4 atomes de carbone et pourvu que, lorsque $XR_1$ représente un méthoxy, R est alors autre que le 2,3-dibromopropyle.

4. Composés tels que revendiqués dans la revendication 1, présentant la formule:

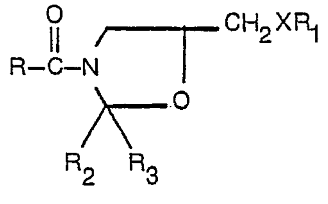

dans laquelle:

X représente un oxygène ou un soufre; et

$R_1$ représente un alkyle contenant de 1 à 6 atomes de carbone;

R est choisi entre un haloalkyle contenant de 1 à 4 atomes de carbone et le terme halo comprend chloro et bromo, et un alkoxycarboalkyle contenant de 3 à 6 atomes de carbone;

$R_2$ est choisi entre phényle, p-chlorophényle et p-bromophényle; et

$R_3$ est choisi entre l'hydrogène et le méthyle.

5. Composés tels que revendiqués dans la revendication 1, choisis entre la 2,2-diméthyl-3-(3-bromopropionyl)5-pentoxyméthyloxazolidine, la 2,2-diméthyl-3-(5-chlorovaléryl)5-isopropoxyméthyloxazolidine, et la 2,2-diméthyl-3-(5-chlorovaléryl)5-allyloxyméthyloxazolidine.

6. Procédé de préparation d'un composé tel que revendiqué dans la revendication 1, caractérisé en ce que qu'il consiste à traiter un intermédiaire de formule:

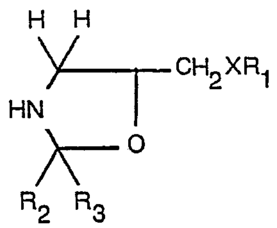

(XI)

dans laquelle:

X, $R_1$, $R_2$ et $R_3$ ont les significations données dans la revendication 1, par l'un des modes opératoires suivants, à savoir:

1. pour la production de composés dans lesquels R représente un haloalkyle ou un alkoxycarboalkyle, l'acylation directe du composé de formule XI;

2. pour la production de composés dans lesquels R représente un alkylthio, l'alkylthioformylation direct d'un composé de formule XI;

3. pour la production de composés dans lesquels R représente le paratoluènesulfonyle, la carbamylation directe du composé de formule XI.

7. Composition herbicide comprenant un herbicide à base de thiolcarbamate et une quantité efficace en tant qu'antidote non phytotoxique d'un composé antidote de ce carbamate, tel que revendiqué dans les revendications 1, 2, 3, 4 ou 5.

8. Procédé pour réduire les dommages causés aux récoltes, ces dommages étant causés par un herbicide à base de thiolcarbamate consistant en une application au sol dans lequel cette récolte doit être plantée et doit pousser, d'une quantité efficace en tant qu'antidote non phytotoxique d'un composé tel que revendiqué dans les revendications 1, 2, 3, 4 ou 5.

9. Procédé pour réduire les dommages causés aux récoltes, ces dommages étant causés par un herbicide à base de thiolcarbamate, consistant à appliquer dans le sillon à la graine et au sol adjacent du sillon ouvert avant sa courverture destinée à achever la plantation, une quantité efficace en tant qu'antidote non phytotoxique d'un composé tel que revendiqué dans les revendications 1, 2, 3, 4 ou 5.

10. Procédé pour protection d'une récolte des dommages dûs à un composé à base de thiolcarbamate actif en tant qu'herbicide consistant à appliquer à la graine avant la plantation une quantité efficace en tant qu'antidote non phytotoxique d'un composé tel que revendiqué dans les revendications 1, 2, 3, 4 ou 5.

**Revendications pour l'Etat contractant: AT**

1. Procédé de préparation de composés de formule:

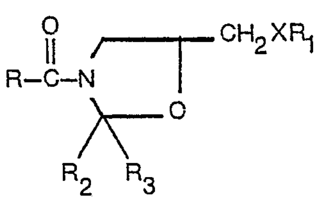

dans laquelle:

$XR_1$ représente un phénoxy;

R est choisi entre un haloalkyle contenant de 1 à 5 atomes de carbone, où halo représente chloro ou bromo, et un p-méthyl phénylsulfonylamido; et

$R_2$ et $R_3$ sont indépendamment choisis parmi les alkyles contenant de 1 à 3 atomes de carbone; ou dans laquelle:

X représente un oxygène ou un soufre;

$R_1$ est choisi entre un alkyle et un alkényle;

R est choisi entre un haloalkyle et un alkylthio; et

$R_2$ et $R_3$ sont indépendamment choisis entre l'hydrogène et un alkyle contenant de 1 à 3 atomes de carbone, pourvu que $R_1 + R_2$ soient inférieurs à ou égaux à 6 atomes de carbone et pourvu encore que, lorsque $XR_1$ représente un thioéthyle, R est alors autre qu'un haloalkyle possédant de 3 à 4 atomes de carbone et pourvu que, lorsque $XR_1$ représente un méthoxy, R est autre que le 2,3-dibromopropyl; ou dans laquelle X représente un oxygène ou un soufre et $R_1$ représente un alkyle contenant de 1 à 6 atomes de carbone; R est choisi entre un haloalkyle contenant de 1 à 4 atomes de carbone, et le terme

halo comprend chloro et bromo et un alkoxycarbonylalkyle contenant de 3 à 6 atomes de carbone; $R_2$ est choisis entre: phényle, p-chlorophényle et p-bromophényle; et $R_3$ ets choisi entre l'hydrogène et le méthyle, consistant à traiter un intermédiaire de formule:

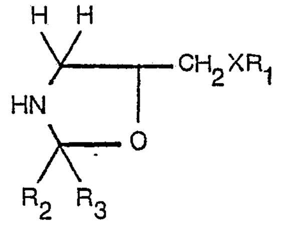

dans laquelle:

X, $R_1$, $R_2$ et $R_3$ ont les significations données ci-dessus; par l'un des modes opératoires suivants, à savoir:

1. pour la production de composés dans lesquels R représente un haloalkyle ou un alkoxycarboalkyle, l'acylation directe du composé de formule XI;

2. pour la production de composés dans lesquels R représente un alkylthio, l'alkylthioformylation· directe d'un composé de formule XI;

3. pour la production de composés dans lesquels R représente un p-toluènesulfonyle, la carbamylation directe du composé de formule XI.

2. Composition herbicide comprenant un herbicide à base de thiolcarbamate et une quantité efficace en tant qu'antidote non phytotoxique d'un composé antidote de ce carbamate de formule I défini dans la revendication 1, et dont la préparation est revendiquée dans la revendication 1.

3. Procédé de réduction des dommages causés aux récoltes, ces dommages étant dûs à un herbicide à base de thiolcarbamate, consistant en une application au sol dans lequel cette récolte doit être plantée et doit pousser, d'une quantité efficace en tant qu'antidote non phytotoxique d'un composé de formule I défini dans la revendication 1.

4. Procédé de réduction de dommages causés aux récoltes, ces dommages étant causés par un herbicide à base de thiolcarbamate consistant à appliquer dans le sillon aux graines et au sol adjacent dans le sillon ouvert avant sa couverture devant destinée à achever la plantation, une quantité efficace en tant qu'antidote non phytotoxique d'un composé de formule I tel que défini dans la revendication 1, et dont la préparation est revendiquée dans la revendication 1.

5. Procédé pour la protection d'une récolte des dommages dûs à un composé de thiolcarbamate herbicide actif consistant à appliquer à la graine avant plantation une quantité efficace en tant qu'antidote non phytotoxique d'un composé de formule I tel que défini dans la revendication 1.

**Patentansprüche für die Vertragsstaaten: BE CH DE FR GB IT LI NL**

1. Verbindungen gemäß der Formel

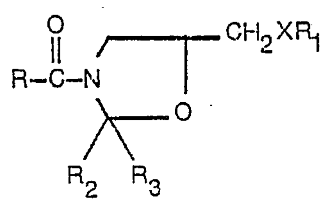

worin $XR_1$ ein Phenoxyrest ist, R ausgewählt ist aus Halogenalkylresten mit 1 bis 5 Kohlenstoffatomen, wobei Halogen Chlor oder Brom bedeutet, und dem p-Methylphenylsulfonylamidorest; und $R_2$ und $R_3$ unabhängig Alkylreste mit 1 bis 3 Kohlenstoffatomen darstellen; oder worin X Sauerstoff oder Schwefel bedeutet, $R_1$ ausgewählt ist aus Alkyl- und Alkenylresten; R ausgewählt ist aus Halogenalkyl- und Alkylthioresten; und $R_2$ und $R_3$ unabhängig ausgewählt sind aus Wasserstoff und Alkylresten mit 1 bis 3 Kohlenstoffatomen, mit der Maßgabe, daß $R_1 + R_2$ weniger als oder gleich 6 Kohlenstoffatome aufweisen, und mit der weiteren Maßgabe, daß dann, wenn $XR_1$ der Thioethylrest ist, R kein Halogenalkylrest mit 3 bis 4 Kohlenstoffatomen ist, und mit der Maßgabe, daß dann, wenn $XR_1$ der Methoxyrest ist, R kein 2,3-Dibrompropylrest ist; oder worin X Sauerstoff oder Schwefel darstellt und $R_1$ ein Alkylrest mit 1 bis 6 Kohlenstoffatomen bedeutet; R ausgewählt ist aus Halogenalkylresten mit 1 bis 4 Kohlenstoffatomen, und der Ausdruck Halogen Chlor und Brom umfaßt, und Alkoxycarbonylalkylresten mit 3 bis 6 Kohlenstoffatomen, $R_2$ ausgewählt ist aus Phenyl-, p-Chlorphenyl- und p-Bromphenylresten; und $R_3$ ausgewählt ist aus Wasserstoff und dem Methylrest.

2. Verbindungen wie in Anspruch 1 beansprucht gemäß der Formel

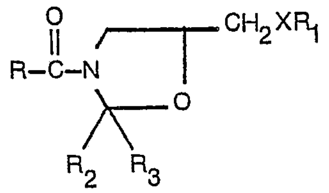

worin $XR_1$ ein Phenoxyrest ist, R ausgewählt ist aus Halogenalkylresten mit 1 bis 5 Kohlenstoffatomen, wobei Halogen Chlor oder Brom bedeutet, und dem p-Methyl-phenylsulfonylamidorest; und $R_2$ und $R_3$ unabhängig ausgewählt sind aus Alkylresten mit 1 bis 3 Kohlenstoffatomen.

3. Verbindungen wie in Anspruch 1 beansprucht gemäß der Formel

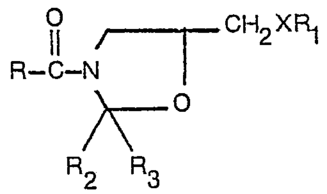

worin X Sauerstoff oder Schwefel darstellt, $R_1$ ausgewählt ist aus Alkyl- und Alkenylresten; R ausgewählt ist aus Halogenalkyl- und Alkylthioresten; und $R_2$ und $R_3$ unabhängig ausgewählt sind aus Wasserstoff und Alkylresten mit 1 bis 3 Kohlenstoffatomen, mit der Maßgabe, daß $R_1 + R_2$ weniger als oder gleich 6 Kohlenstoffatome aufweisen, und mit der weiteren Maßgabe, daß dann, wenn $XR_1$ der Thioethylrest ist, R kein Halogenalkylrest mit 3 oder 4 Kohlenstoffatomen ist, und mit der Maßgabe, daß dann, wenn $XR_1$ der Methoxyrest ist, R kein 2,3-Dibrompropylrest ist.

4. Verbindungen wie in Anspruch 1 beansprucht gemäß der Formel

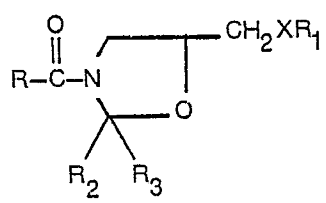

worin X Sauerstoff oder Schwefel bedeutet und $R_1$ ein Alkylrest mit 1 bis 6 Kohlenstoffatomen ist; R ausgewählt ist aus Halogenalkylresten mit 1 bis 4 Kohlenstoffatomen, und der Ausdruck Halogen Chlor und Brom umfaßt, und Alkoxycarboalkylresten mit 3 bis 6 Kohlenstoffatomen; $R_2$ ausgewählt ist aus Phenyl-, p-Chlorphenyl- und p-Bromphenylresten; und $R_3$ ausgewählt ist aus Wasserstoff und dem Methylrest.

5. Verbindungen wie in Anspruch 1 beansprucht, ausgewählt aus 2,2-Dimethyl-3-(3-bromopropionyl)5-pentoxymethyl-oxazolidin, 2,2-Dimethyl-3(5-chlorvaleryl)5-isopropoxymethyloxazolidin und 2,2-Dimethyl-3(5-chlorvaleryl)5-allyloxymethyl-oxazolidin.

6. Verfahren zur Herstellung einer Verbindung wie in Anspruch 1 beansprucht, dadurch gekennzeichnet, daß es die Behandlung eines Zwischenproduktes der Formel

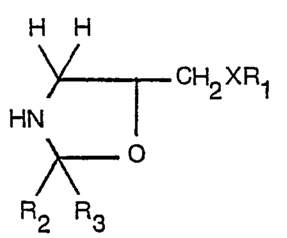

(XI)

worin X, $R_1$, $R_2$ und $R_3$ die in Anspruch 1 angegebenen Bedeutungen aufweisen, nach einem der folgenden Verfahren umfaßt, nämlich:

1. zur Herstellung von Verbindungen, worin R ein Halogenalkyl- oder Alkoxycarboalkylrest ist, direkte Acylierung der Verbindung der Formel XI;

2. zur Herstellung von Verbindungen, worin R ein Alkylthiorest ist, direkte Alkylthioformylierung einer Verbindung der Formel XI;

3. Zur Herstellung von Verbindungen, worin R der p-Toluolsulfonylrest ist, direkte Carbamylierung der Verbindung der Formel XI.

7. Herbicide Zusammensetzung, enthaltend ein Thiolcarbamat-Herbicid und eine nichtphytotoxische, antidotisch wirksame Menge einer Antidotverbindung dafür wie in Anspruch 1, 2, 3, 4 oder 5 beansprucht.

8. Verfahren zur Verringerung von Beschädigungen an Nutzpflanzenkulturen, wobei diese Beschädigungen auf ein Thiolcarbamat-Herbicid zurückzuführen sind, umfassend die Anwendung einer nichtphytotoxischen, antidotisch wirksamen Menge einer Verbindung wie in Anspruch 1, 2, 3, 4 oder 5 beansprucht auf den Boden, in welchem diese Nutzpflanzenkultur gesät bzw. gepflanzt werden und wachsen soll.

9. Verfahren zur Verringerung von Beschädigungen an Nutzpflanzenkulturen, wobei diese Beschädigungen auf ein Thiolcarbamat-Herbicid zurückzuführen sind, welches die Anwendung einer nichtphytotoxischen, antidotisch wirksamen Menge einer Verbindung wie in Anspruch 1, 2, 3, 4 oder 5 beansprucht in die Saatfurche auf den Samen und den benachbarten Boden in der offenen Furche vor dem Bedecken unter Erzielung eines eingesäten bzw. bepflanzten Zustandes umfaßt.

10. Verfahren zum Schützen einer Pflanzenkultur vor Beschädigungen, die auf eine wirksame herbicide Thiolcarbamatverbindung zurückführen sind, umfassend die Anwendung einer nichtphytotoxischen, antidotisch wirksamen Menge einer Verbindung wie in Anspruch 1, 2, 3, 4 oder 5 beansprucht auf den Pflanzensamen vor dem Säen bzw. Pflanzen.

**Patentansprüche für den Vertragsstaat: AT**

1. Verfahren zur Herstellung von Verbindungen der Formel:

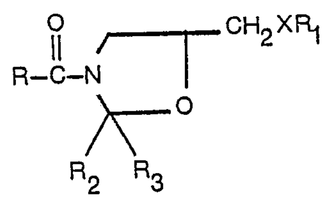

worin $XR_1$ einen Phenoxyrest darstellt, R ausgewählt ist aus Halogenalkylresten mit 1 bis 5 Kohlenstoffatomen, wobei Halogen Chlor oder Brom bedeutet, und dem p-Methyl-phenylsulfonylamidorest; und $R_2$ und $R_3$ unabhängig ausgewählt sind aus Alkylresten mit 1 bis 3 Kohlenstoffatomen; oder worin X Sauerstoff oder Schwefel bedeutet, $R_1$ ausgewählt ist aus Alkyl- und Alkylengruppen; R ausgewählt ist aus Halogenalkyl und Alkylthioresten; und $R_2$ und $R_3$ unabhängig ausgewählt sind aus Wasserstoff und Alkylresten mit 1 bis 3 Kohlenstoffatomen, mit der Maßgabe, daß $R_1$ und $R_2$ weniger als oder gleich 6 Kohlenstoffatome aufweisen, und mit der weiteren Maßgabe, daß dann, wenn $XR_1$ den Thioethylrest bedeutet, R kein Halogenalkylrest mit 3 oder 4 Kohlenstoffatomen ist, und mit der Maßgabe, daß dann, wenn $XR_1$ den Methoxyrest darstellt, R kein 2,3-Dibromopropylrest ist; oder worin X Sauerstoff oder Schwefel bedeutet und $R_1$ ein Alkylrest mit 1 bis 6 Kohlenstoffatomen ist; R ausgewählt ist aus Halogenalkylresten mit 1 bis 4 Kohlenstoffatomen, und der Ausdruck Halogen Chlor und Brom umfaßt, und Alkoxy-carbonylalkylresten mit 3 bis 6 Kohlenstoffatomen, $R_2$ ausgewählt ist aus dem Phenyl-, p-Chlorphenyl- und p-Bromphenylrest; und $R_3$ ausgewählt ist aus Wasserstoff und dem Methylrest, welches die Behandlung eines Zwischenproduktes der Formel

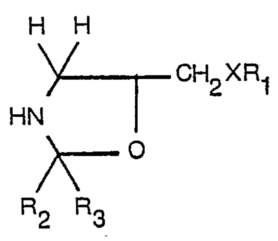

(XI)

worin X, $R_1$, $R_2$ und $R_3$ die vorstehenden Bedeutungen aufweisen, nach einem der folgenden Verfahren umfaßt, nämlich:

1. zur Herstellung von Verbindungen, in denen R ein Halogenalkyl- oder Alkoxycarboalkylrest ist, direkte Acylierung der Verbindung der Formel XI;

2. zur Herstellung von Verbindungen, in denen R ein Alkylthiorest ist, direkte Alkylthioformylierung einer Verbindung der Formel XI;

3. zur Herstellung von Verbindungen, in denen R der p-Toluolsulfonylrest ist, direkte Carbamylierung der Verbindung der Formel XI.

2. Herbicide Zusammensetzung, umfassend ein Thiolcarbamat-Herbicid und eine nichtphytotoxische, antidotisch wirksame Menge einer Antidotverbindung däfur mit der Formel I, wie in Anspruch definiert, deren Herstellung in Anspruch 1 beansprucht ist.

3. Verfahren zur Verringerung von Beschädigungen an Nutzpflanzenkulturen, wobei diese Beschädigungen auf ein Thiolcarbamat-Herbicid zurückzuführen sind, umfassend die Anwendung einer nichtphytotoxischen, antidotisch wirksamen Menge einer Verbindung der Formel I wie in Anspruch 1 definiert auf den Boden, in dem diese Nutzpflanzenkultur gesät bzw. gepflanzt werden und wachsen soll.

4. Verfahren zur Verringerung von Beschädigungen an Nutzpflanzenkulturen, wobei diese Beschädigungen auf ein Thiolcarbamat-Herbicid zurückzuführen sind, welches die Anwendung einer nichtphytotoxischen, antidotisch wirksamen Menge einer Verbindung der Formel I wie in Anspruch 1 definiert, deren Herstellung in Anspruch 1 beansprucht ist, in der Saatfurche auf den Samen und den benachbarten Boden in der offenen Furche vor dem Bedecken unter Erzielung eines eingesäten bzw. bepflanzten Zustandes umfaßt.

5. Verfahren zum Schutz einer Pflanzenkultur vor Beschädigungen, die auf eine aktive herbicide Thiolcarbamatverbindung zurückführen sind, umfassend die Anwendung einer nichtphytotoxischen, antidotisch wirksamen Menge einer Verbindung der Formel I wie in Anspruch 1 definiert, auf den Pflanzensamen vor dem Pflanzen bzw. Säen.